(19) **Europäisches Patentamt European Patent Office Office européen des brevets**

(11) **EP 4 251 027 B1**

(12) **EUROPEAN PATENT SPECIFICATION**

(45) Date of publication and mention of the grant of the patent:
**12.11.2025 Bulletin 2025/46**

(21) Application number: **21810056.8**

(22) Date of filing: **24.11.2021**

(51) International Patent Classification (IPC):
**A61B 5/00** (2006.01)     **A61B 8/08** (2006.01)

(52) Cooperative Patent Classification (CPC):
**A61B 8/08; A61B 5/0031; A61B 5/6867;**
A61B 5/076; A61B 8/0875; A61B 8/0883;
A61B 8/12; A61B 8/485; A61B 8/5207;
A61B 8/5223; A61B 8/58; G10K 11/162;
G10K 11/165

(86) International application number:
**PCT/EP2021/082744**

(87) International publication number:
**WO 2022/112279 (02.06.2022 Gazette 2022/22)**

(54) **IMPLANTABLE SENSOR**

IMPLANTIERBARER SENSOR

CAPTEUR IMPLANTABLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.11.2020 EP 20210051**

(43) Date of publication of application:
**04.10.2023 Bulletin 2023/40**

(73) Proprietor: **ETH Zurich**
**8092 Zurich (CH)**

(72) Inventors:
• **HIEROLD, Christofer**
**5400 Baden (CH)**
• **ROMAN, Cosmin**
**8907 Wettswil (CH)**
• **MAINI, Lucrezia**
**8032 Zürich (CH)**

• **FALK, Volkmar**
**10587 Berlin (DE)**
• **CESAROVIC, Nikola**
**8050 Zürich (CH)**

(74) Representative: **Piticco, Lorena**
**Isler & Pedrazzini AG**
**Giesshübelstrasse 45**
**Postfach 1772**
**8027 Zürich (CH)**

(56) References cited:
**US-A1- 2005 049 499     US-A1- 2020 359 897**

• **BORNER M W ET AL: "Ultrasonic measurements with micromembranes", SENSORS AND ACTUATORS A: PHYSICAL, ELSEVIER BV, NL, vol. 46, no. 1-3, 1 January 1995 (1995-01-01), pages 62 - 65, XP027205901, ISSN: 0924-4247, [retrieved on 19950101]**

**Description**

TECHNICAL FIELD

The present invention relates to an implantable sensor for determining at least one parameter according to claim 1, an implant comprising such an implantable sensor according to claim 11, an assembly for determining at least one parameter comprising such an implantable sensor according to claim 12, a method of determining at least one parameter using such an implantable sensor or an assembly according to claim 13, a method of producing an implantable sensor for determining at least one parameter according to claim 14, as well as to a computer program product that causes the determination of the at least one parameter according to claim 15.

PRIOR ART

The determination of physical parameters is common and widespread in medical technology in order to monitor events in the human or animal body. For example, US 2020/0015678 A1 discloses an intraocular pressure sensor that comprises a stretchable inductor in contact with an eye of a human being for glaucoma detection and monitoring. The sensor is based on an inductor which is a passive strain gauge and which is connected to a capacitor in a resonant circuit. The sensor interrogation is achieved via a receiver being installed on glasses that are worn by the human being. US 10 595 771 B2 discloses a strain sensor for bone healing assessment that comprises an implant for attachment to the bone and two sensors. One of the sensors is used for measuring the strain on the injured section of the bone and the other sensor is used as a reference. The sensors are MEMS RF devices, wherein a frequency shift caused by a change of the capacitance in the sensor is associated with different values of strain. From US 10 105 103 B2 an atrial pressure implant is known, which implant is located in the left atrium and is constituted of an antenna unit whose components are a ferrite core and a coil, a pressure sensor and a dedicated circuitry for noise and drift compensations. The antenna communicates with an external unit in the form of a wearable belt by inductive coupling.

From US 2020/359897 A1 systems and a method to measure pressure are known, wherein the system includes a force sensor can that be implanted into a patient to measure, for example, cardiac pressure. The force sensor includes first and second film layers that define a plurality of pressure cells. An external pressure deforms the pressure cells and changes their resonant frequency.

Börner M. W. et al. ("Ultrasonic measurements with micromembranes", SENSORS AND ACTUATORS A: PHYSICAL, ELSEVIER BV, NL, vol. 46, no. 1-3, 1 January 1995 (1995-01-01) pages 62-65, XP027205901, ISSN: 0924-4247) discloses contactless ultrasonic measurements of physical properties by sensing the echo of ultrasonic sound from an array of micromembranes.

From US 2005/049499 A1 a protected resonating sensor is known that includes resonating sensor units having vibratable members. The protected sensor includes a compliant member that forms part of one or more chambers. A first side of the compliant member is exposed to a medium in a measurement environment. The sensor unit is a resonating sensor unit having a resonance frequency that depends on the value of a physical variable in the measurement environment. The protected sensor includes a substantially non-compressible medium disposed within the chamber(s).

A majority of these devices are based on electromagnetic coupling and suffer from several disadvantages. For example, they are not safe for continuous monitoring in humans and they often exhibit a limited sensor performance such as a limited penetration depth. Furthermore, these kind of devices require a dedicated receiver that is designed and customized for the particular application of the device. Moreover, complex electronic systems and fabrication processes are typically involved as well.

SUMMARY OF THE INVENTION

It is an object of the present invention to overcome the drawbacks of the prior art. In particular, it is an object to provide an implantable sensor that allows a safe and at the same time practical determination of at least one parameter.

This object is achieved with an implantable sensor according to claim 1. In particular, an implantable sensor for determining at least one parameter, in particular a physical parameter, is provided, wherein the implantable sensor comprises a sensor body. The sensor body is responsive to the at least one parameter and is configured to yield at least one response signal upon an interrogation of the sensor body with at least one interrogation signal comprising acoustic waves. The at least one response signal is associated with the at least one parameter. The sensor body comprises or consists of at least one material having an ordered structure. The at least one response signal is associated with one or more bulk effects of the at least one material having an ordered structure.

The expression "the sensor body is responsive to the at least one parameter" means that an interaction between the sensor body and the parameter takes place when the sensor body is subjected to the parameter. As such, the response signal that is yielded by the sensor body is affected by the parameter, as well. Hence, by evaluating or assessing the response signal that is yielded by the sensor body the parameter can be determined.

As just mentioned, the sensor body comprises or consists of at least one material having an ordered structure, and wherein said material having an ordered structure is configured for an interrogation with acoustic waves. In other words, the implantable sensor can be interrogated with an interrogation device that is based on acoustic waves, in particular ultrasound waves, and which is thus safe for a long-term monitoring in-vivo. Furthermore, since the material having an ordered structure is interrogated, there is no need for external powering or communication systems. Instead, a passive and wireless interrogation can be performed. The implantable sensor according to the invention therefore enables a safe and at the same time practical determination of the parameter.

The material having an ordered structure comprises an acoustic band structure, and wherein said acoustic band structure is configured to exhibit a change when the sensor body is subjected to the parameter. In other words, when the sensor is subjected to the parameter the parameter can change the acoustic band structure of the material having an ordered structure.

The acoustic band structure of the material having an ordered structure is well-known to the skilled person and corresponds to a number of frequency bands and possibly also frequency gaps. The frequency bands correspond to the discrete eigenvalues, i.e. resonance modes, of the material having an ordered structure. Said modes are mainly part of the acoustic branch of the dispersion relation of the material having an ordered structure. The dispersion relation constrains the spatial frequency and the wavelength such, that for any wavelength of the interrogation signal only a discrete set of allowed frequencies at which the acoustic waves can travel in the material having an ordered structure are accessible. However, it is important to note that the frequency bands can likewise be provided by continuous states. In this case, a frequency of the interrogation signal can take any value within the frequency band in the material having an ordered structure.

In any case, a change in the acoustic band structure corresponds to a displacement or scaling of the acoustic bands. In fact, the acoustic bands are shifted in frequency, wherein an upward shift or downward shift is possible, but also a change in the band width is possible. For example, and as will be explained further below, the parameter interacting with the sensor body could correspond to strain that is applied to the sensor body. In this case, the amount of strain will determine the shift or scaling of the acoustic bands. The strain will furthermore determine whether the bands will shift upwards or downwards in frequency. As an example, if the strain is associated with a positive value, i.e. when the sensor body is undergoing tensile strain or a positive strain, the bands will shift downwards. A downwards shift is understood as a shift of the bands towards lower frequencies. In this same example, if the strain is associated with a negative value, i.e. when the sensor body is undergoing a compressive strain or a negative strain, the bands will shift upwards. An upwards shift is understood as a shift towards higher frequencies. More generally, both the direction of the shift and the magnitude depend on the particular structural details of the material having an ordered structure and the type of the deformation or strain being applied.

The material having an ordered structure can furthermore comprise an acoustic band gap. Said acoustic band gap corresponds to a gap between two acoustic bands of an acoustic band structure and is associated with a range or interval of frequencies at which acoustic waves cannot travel through the material having an ordered structure. A change in the acoustic band structure modulates the acoustic band gap accordingly. In this context it should be noted that a change in the bandgap does not necessarily vary linearly with respect to the strain applied. Instead, some acoustic modes could be more sensitive upon application of strains than others.

Thus, it is conceivable that the implantable sensor allows the detection of changes in the acoustic band structure as well as changes in the acoustic band gap, and wherein the parameter such as strain is determinable from said changes.

Additionally or alternatively, the material having an ordered structure comprises a lattice and/or a lattice-like structure, and wherein said lattice and/or lattice-like structure is distorted when the sensor body is subjected to the parameter.

Within the context of the present invention a lattice is understood as a structure comprising a periodic or regular arrangement of its constituents. As will be explained in greater detail further below said constituents can correspond to substructures or voids being arranged in a substrate such as a matrix. Within the context of the present invention a lattice-like structure is understood as a structure comprising a periodic or aperiodic arrangement of its constituents. As will be explained in greater detail further below said constituents can correspond to substructures or voids being arranged in a substrate such as a matrix.

Thus, the material having an ordered structure can comprise or consist of a lattice and/or a lattice-like structure, and wherein the parameter can distort said lattice and/or said lattice-like structure. When the lattice and/or the lattice-like structure is distorted, one or more lattice constants of the lattice and/or of the lattice-like structure are changed accordingly. In other words, the lattice constants are configured to exhibit a change when the sensor body is subjected to the parameter.

The lattice constants correspond to a dimension of the smallest cell or substructure in a lattice or lattice-like structure, which is periodically repeated.

Said change of the acoustic band structure and of the acoustic band gap and said distortion of the lattice and/or of the lattice-like structure are called bulk effects. In other words, the interaction of the sensor body with the parameter results in bulk effects that are generated in the sensor body. Because the interrogation signal is irradiated onto the sensor body and because the sensor body, upon such an irradiation, yields a response signal, said response signal bears the impact of the bulk effects. It can therefore be said that the change of the acoustic band structure and/or of the acoustic band gap and/or the distortion of the lattice and/or the distortion of the lattice-like structure correspondingly changes the response signal as well. An analysis or assessment of the response signal therefore enables the determination of the parameter, see also further below.

The material having an ordered structure is preferably configured to exhibit a deformation when subjected to the parameter, said deformation preferably being a one-dimensional or a multi-dimensional spatial deformation along one or more spatial directions and/or a shear deformation.

That is, the material having an ordered structure can be configured so as to spatially deform upon an interaction of the sensor body with the parameter. To this end a one-dimensional deformation along one spatial direction or a multi-dimensional deformation along two or more spatial directions, including shear deformation, are conceivable. Said spatial deformation preferably corresponds to an elongation or a compression of the sensor body along one or more spatial directions. Depending on the dimensionality of the deformation said deformation can also be seen as a change in size or volume of the sensor body. In other words, the spatial deformation preferably corresponds to a deformation with respect to a (fictitious) fixed point of the sensor body. That is, a distance between one or more (fictitious) points of the sensor body and said fixed point is preferably changed during a deformation. Again in other words it is preferred that the spatial deformation along a spatial direction does not correspond to a rigid body displacement.

Additionally or alternatively said deformation preferably corresponds to a shear deformation. In the present context a shear deformation is understood as a deformation of the material having an ordered structure in which an angle between (fictitious) originally perpendicular lines of the material having an ordered structure is changed. To this end it is particularly preferred that the material having an ordered structure can exhibit combinations of any spatial deformation along any one or more spatial directions and shear deformations, such as a torsional deformation. That is to say, the material having an ordered structure is preferably configured to exhibit any deformation obeying a 3x3 dimensional strain tensor. The deformation(s) particularly preferably correspond to the distortion of the lattice and/or of the lattice-like structure as mentioned above.

The material having an ordered structure preferably exhibits one or more acoustic properties, and wherein said one or more acoustic properties are configured to change when the sensor body is subjected to the parameter. The acoustic properties preferably are an acoustic impedance of the material having an ordered structure and/or at least one mechanical property of the material having an ordered structure such as the Young's modulus of the material having an ordered structure and/or the density of the material having an ordered structure and/or an acoustic band structure of the material having an ordered structure and/or a band gap of the material having an ordered structure. That is to say, the material having an ordered structure preferably exhibits one or more acoustic properties, which are changed upon an interaction of the sensor body with the parameter. Changes of the acoustic properties therefore correspond to changes in the acoustic impedance of the material having an ordered structure, changes in the Young's modulus of the material having an ordered structure, changes in the density of the material having an ordered structure, changes in the acoustic band structure of the material having an ordered structure, or changes in the band gap of the material having an ordered structure. Here, it should be noted that these parameters, i.e. acoustic impedance, the Young's modulus and the density, can have an impact on the acoustic band structure such that their change influences the acoustic band structure. However, even if these parameters remain constant the acoustic band structure can still be affected by a deformation such as a simple geometrical distortion, e.g. a spatial deformation.

Moreover, one or more of these changes in the acoustic properties, in particular a change in the acoustic band structure and/or a change in the acoustic band gap of the material having an ordered structure, can result in a change of the reflectance and/or transmittance of the material having an ordered structure. In other words, a change in the acoustic band structure can be observed in, in particular can be determined from, a transmission spectrum and/or from a reflection spectrum. Likewise, a change in the acoustic band gap can be observed in, in particular can be determined from, a transmission spectrum and/or from a reflection spectrum. Consequently, said changes in the reflectance or in the transmittance are preferably observed by changes in a response signal corresponding to a reflection spectrum and/or in a response signal corresponding to a transmission spectrum and/or in an interrogation signal resulting in a particular response signal in the form of a reflection spectrum and/or in an interrogation signal resulting in a particular response signal in the form of a transmission spectrum, see also below.

The at least one parameter is preferably determinable from a change in the response signal, said change preferably being a spectral change, particularly preferably a change of the intensity of the response signal and/or a change of the amplitude of the response signal and/or a phase change of the response signal and/or a modulation of the response signal.

Additionally or alternatively, the at least one parameter is preferably determinable from a change of the interrogation signal that yields a particular response signal, said particular response signal preferably being a signal that is constant over time.

That is, the parameter can be determined in different ways. Namely, on the one side it is conceivable that the parameter is determined from a change in the response signal. This mode of operation is referred to as open-loop operation. In the open-loop operation the interrogation signal, in particular a frequency of the interrogation signal and/or a bandwidth of the interrogation signal, is preferably kept constant. A constant frequency is understood as a fixed frequency, i.e. a single frequency that does not change over time. A constant bandwidth of the interrogation signal is understood as two or more frequencies that do not change over time. Depending on the source providing the interrogation signal said bandwidth can be in the range of 2-3 MHz, for example. Thus, said bandwidth can be referred to as narrow-band. The at least one parameter is preferably determinable by a comparison and/or a correlation of the response signal, in particular of the change in the response signal, with one or more table values and/or one or more calibration values. On the other side it is conceivable that the parameter is determined from a change of the interrogation signal. Said change of the interrogation signal preferably is a change of a frequency of the interrogation signal and/or a change of the intensity of the interrogation signal and/or a change of the amplitude of the interrogation signal and/or a phase change of the interrogation signal and/or a modulation of the interrogation signal. This mode of operation is referred to as closed-loop operation. In the closed-loop operation the interrogation signal, in particular a frequency of the interrogation signal, is tuned so as to obtain a response signal, in particular a transmission and/or a reflection of the response signal that remains the same, i.e. that is constant over time. The at least one parameter is preferably determinable by a comparison and/or a correlation of the interrogation signal, in particular of the change of the interrogation signal, with one or more table values and/or one or more calibration values. In particular, in the closed-loop operation a frequency of the interrogation signal is preferably continuously adjusted, i.e. changed, such that the response signal maintains a constant intensity and/or a constant amplitude and/or a constant phase. For example, if the parameter to be determined is strain an interrogation signal at a first strain, e.g. a strain of zero, yields a particular first transmission response signal and/or a particular first reflection response signal. Upon the application of a second strain being different from the first strain the sensor body yields a particular second transmission response signal and/or a particular second reflection response signal. Hence, in the closed-loop operation the interrogation signal is changed or adjusted such that the first transmission response signal corresponds to the second transmission response signal and/or such that the first reflection response signal corresponds to the second reflection response signal. In other words, the interrogation signal, in particular a frequency of the interrogation signal is changed or adjusted such that a value of the reflectance and/or of the transmittance does not change. Again in other words, the parameter such as strain can modulate the reflectance and/or the transmittance of the sensor body, and wherein this modulation is taken into account by correspondingly modulating the interrogation signal. Hence, it can be said that the at least one interrogation signal can be associated with the at least one parameter, as well.

In any case it is preferred that the response signal comprises bulk waves and/ or bulk acoustic waves and/or waves being reflected from a bulk of the material having an ordered structure and/or waves being transmitted through the bulk of the material having an ordered structure. In other words, the sensor body is preferably configured such that bulk waves and/ or bulk acoustic waves and/or waves being reflected from a bulk of the material having an ordered structure and/or waves being transmitted through the bulk of the material having an ordered structure are generated upon an irradiation of the sensor body with acoustic waves. Waves being reflected or transmitted from the bulk of the material having an ordered structure preferably correspond to acoustic waves that are elastically scattered from the sensor body and are herein called bulk waves. Consequently, it is preferred that the sensor body is configured to at least partially transmit the interrogation signal, whereby a transmission spectrum constituting the response signal is generated. Additionally or alternatively it is preferred that the sensor body is configured to at least partially reflect the interrogation signal, whereby a reflection spectrum constituting the response signal is generated. However, it is likewise conceivable that the acoustic waves excite the sensor body such that bulk acoustic waves are generated by the interrogation signal. To this end it should be noted that an interaction between the interrogation signal and the entire bulk of the material having an ordered structure is conceivable as well as an interaction between the interrogation signal and only a part of the bulk of the material having an ordered structure is conceivable. In the latter case the interaction preferably takes place between one or more surface states or surface bands of the bulk. Whether an interaction with the entire bulk of the material having an ordered structure or only with a part of the bulk of the material having an ordered structure takes place depends on the penetration depth of the interrogation signal. Said penetration depth can be adjusted or selected by an operator, for example by tuning a frequency of the interrogation signal, wherein higher frequencies result in a lower penetration depth and lower frequencies result in a higher penetration depth.

As already mentioned previously, the material having an ordered structure can comprise a lattice and/or a lattice like structure. Said lattice and/or lattice-like structure is preferably constituted by one or more cells, and wherein a length of one cell with respect to a spatial direction is preferably in the order of magnitude of the speed of sound within the material having an ordered structure divided by a frequency of the interrogation signal or a frequency of the response signal. That is, the material having an ordered structure can be described in terms of cells as it is common in the field of crystallography. In fact, a cell of said material having an ordered structure is defined here as the smallest periodically repeating unit.

In other words the material having an ordered structure can be understood as being or an ordered or regular or systematic arrangement of one or more cell types, wherein the expressions "ordered" or "regular" or "systematic" mean periodic or aperiodic as defined above. In other words, if the material having an ordered structure is periodic, there is one repeating cell type. If the material having an ordered structure is aperiodic, there are two or more repeating cell types. An example of such a material having an aperiodic structure is a Penrose tiling formed by the aperiodic repetition of two different cell types.

The geometry of the cell, in particular the lattice constants, was found to enable an adjustment or determination of the characteristics of the material having an ordered structure according to the invention.

In fact, by approximating the length l of one cell along one spatial direction with an integer number of (1/2) of the wavelength $\lambda$ the following equation illustrates the relationship existing between the length l of said one cell, the speed of sound c within the material having an ordered structure, and the frequency f of the interrogation signal or of the response signal:

$$l \sim n\frac{\lambda}{2} = n\,\frac{c}{2f} \qquad (1)$$

That is, the length of the cell with respect to one spatial direction can be determined by the speed of sound within the material having an ordered structure divided by a frequency of the interrogation signal or divided by a frequency of the response signal. In this regard it should be noted that a frequency of the response signal equals a frequency of the interrogation signal, both in the closed-loop operation and the open-loop operation.

Hence, by adjusting the length of the cell, or more broadly speaking by adjusting a lattice constant, a frequency of the interrogation signal that is necessary for interrogating the sensor, i.e. necessary for the sensor body to yield the response signal, can be selected. Likewise, by adjusting a length of the cell, or more broadly speaking by adjusting a lattice constant, a frequency of the response signal can be selected.

Regarding a size of the sensor body it is noted that the sensor body is preferably constituted by at least 1 cell, or by at least 100 cells, or by at least 1000 cells. Additionally or alternatively it is preferred that the sensor body comprises between 1 cell and 1000 cells.

As explained initially the sensor according to the invention is preferably interrogated with an interrogation signal comprising acoustic waves. Consequently, it is preferred that a length of the cell or one or more lattice constants of the material having an ordered structure are such that the interrogation signal corresponds to acoustic waves having one or more frequencies in the range of about 0.5 MHz to 30 MHz, preferably in the range of about 1 MHz to 15 MHz.

In order to enable an interrogation with such an interrogation signal it is preferred that a length of one cell with respect to a spatial direction is in the range of 1 micrometer to 10 millimeter, more preferably in the range of 10 micrometer to 1 millimeter, and/or at least 1 micrometer, more preferably at least 10 micrometer, and/or up to 10 millimeter, more preferably up to 1 millimeter.

The spatial direction preferably is a spatial direction of an arbitrary coordinate system. It is particularly preferred that said spatial direction is parallel to the direction of the k vector of the interrogation signal being incident on the sensor body.

The material having an ordered structure preferably corresponds to a material having a periodic structure and/or to a material having an aperiodic structure.

A periodic structure is understood as a translational repetition of a unit cell.

An aperiodic structure is understood as a repetition of two or more cells that does not possess translational periodicity.

The material having an ordered structure preferably comprises or consists of a so-called metamaterial and/or one or more biocompatible materials and/or one or more unreactive materials and/or one or more materials having a high resistance to corrosion and/or one or more materials having a shape memory and/or one or more materials being elastic and/or one or more materials being visco-elastic and/or one or more polymers and/or one or more copolymers and/or one or more metals and/or one or more alloys and/or one or more gases.

A preferred metamaterial comprises or consists of an acoustic metamaterial.

Non-limiting examples of conceivable polymers are silicon-based polymers such as polymers comprising siloxane, e.g. Polysiloxane or Polydimethylsiloxane (PDMS), and/or thermoplastic polymers such as Poly(methyl methacrylate) (PMMA), and/or thermosetting polymers, and/or polymers of imide monomers, and/or thermosetting polyimides such as Polyimide, and/or epoxy-based polymers such as SU-8, and/or aromatic hydrocarbon polymers such as Polystyrene (PS), and/or polymers comprising a backbone comprising para-benzenediyl rings connected by 1, 2-ethanediyl bridges such as Parylene, in particular Parylene-C.

Preferred copolymers are copolymers of these polymers. Preferred metals are noble metals, for example platinum, and/or transition metals, for example titanium. Preferred alloys are metal alloys, for example cobalt-chrome (Co-Cr) or Nitinol, i.e. nickel-titanium. A preferred gas is air and/or one or more components of air such as nitrogen or oxygen and/or nobles gases such as argon.

The material having an ordered structure preferably comprises at least one matrix and one or more substructures being arranged within the at least one matrix.

An acoustic impedance of the matrix preferably differs from the acoustic impedance of the substructure(s). Additionally or alternatively a distance among the substructures is preferably about 0.5 micrometer or larger, preferably in the range of about 0.5 micrometer to 9 millimeter, more preferably in the range of about 1 micrometer and 5 millimeter. Additionally or alternatively a cross-section of an individual substructure preferably is about 0.1 micrometer or larger, preferably in the range of about 1 micrometer to 100 micrometer.

That is, it is preferred that the material having an ordered structure comprises a matrix and substructures, and wherein the matrix and the substructures are constituted of two or more different materials. According to the value of their acoustic impedance it is furthermore preferred that said two or more different materials give rise to an acoustic contrast. An acoustic contrast, i.e. a ratio between the acoustic impedance of the matrix and the substructure preferably is at least 1.5 and/or at most 0.67.

The matrix and the particular arrangement of the substructures therein is understood herein as the material having an ordered structure.

It is furthermore conceivable that the material having an ordered structure comprises two or more substructures being the same with regard to their acoustic impedance and/or dimension and/or distance among them, etc. However, it is likewise conceivable that the material having an ordered structure comprises two or more substructures having different acoustic impedances and/or shapes and/or dimensions and/or distances among them, etc.

An acoustic impedance of the matrix and/or of the substructure preferably takes into account the range of acoustic impedance of the human body. An acoustic impedance of the matrix can be lower than or higher than or equal to an acoustic impedance of the human or animal body, preferable of the same or similar acoustic impedance of the human or animal body. An acoustic impedance of the one or more substructures can be lower than or higher than an acoustic impedance of the matrix and/or preferable of the same or similar acoustic impedance of the matrix.

An acoustic impedance of the matrix preferably is in the range of about 0.05 MRayl to 100 MRayl, more preferably in the range of about 2 MRayl to 70 MRayl. A minimal acoustic impedance of the matrix preferably is 0.5 MRayl or more, preferably 2 MRayl or more. A maximal acoustic impedance of the matrix preferably is 100 MRayl or less, preferably 70 MRayl or less.

An acoustic impedance of the substructure preferably is in the range of about 0.0003 MRayl to 100 MRayl, more preferably in the range of about 0.0003 MRayl to 70 MRayl. A minimal acoustic impedance of the substructure preferably is 0.0003 MRayl or more, preferably at least 0.0004 MRayl or more. A maximal acoustic impedance of the substructure preferably is 100 MRayl or less, preferably 70 MRayl or less.

It is furthermore preferred that the matrix comprises or consists of the one or more biocompatible materials and/or the one or more unreactive materials and/or the one or more materials having a high resistance to corrosion and/or the one or more materials having a shape memory and/or the one or more materials being elastic and/or the one or more materials being visco-elastic and/or the one or more polymers and/or the one or more copolymers and/or the one or more metals and/or the one or more alloys and/or the one or more gases mentioned above.

It is furthermore preferred that the one or more substructures comprises or consists of the one or more biocompatible materials and/or the one or more unreactive materials and/or the one or more materials having a high resistance to corrosion and/or the one or more materials having a shape memory and/or the one or more materials being elastic and/or the one or more materials being visco-elastic and/or the one or more polymers and/or the one or more copolymers and/or the one or more metals and/or the one or more alloys and/or the one or more gases mentioned above.

It is furthermore preferred that the matrix and the one or more substructures constitute a metamaterial.

It is furthermore preferred that the matrix exhibits one or more acoustic properties, and wherein said one or more acoustic properties are configured to change when the sensor body is subjected to the parameter. Said acoustic properties preferably is an acoustic impedance of the matrix and/or at least one mechanical property of the matrix such as the Young's modulus of the matrix and/or a density of the matrix.

It is furthermore preferred that the at least one substructure exhibits one or more acoustic properties, and wherein said one or more acoustic properties are configured to change when the sensor body is subjected to the parameter. Said acoustic properties preferably is an acoustic impedance of the substructure and/or at least one mechanical property of the substructure such as the Young's modulus of the substructure and/or a density of the substructure.

By specifically selecting these components a material having an ordered structure of the sensor body is produced which has specific properties, in particular a specific acoustic impedance and/or a specific transmittance and/or a specific reflectance.

The following table lists some possible combinations of these components for illustrative purposes. The expression "High Z" refers to a matrix or a substructure that has a high acoustic impedance. The expression "Low Z" refers to a matrix or substructure that has a low acoustic impedance. The expression "Grey" refers to the choice of a matrix which, in combination with a low or high acoustic impedance for the substructure, generates a transmittance in the order of 0.5 (50%). Moreover, the material combination can include a further distinction between a black sensor body configuration and a white sensor body configuration, respectively. In the black sensor body configuration the combination of the components which constitute the matrix and the substructures is chosen such that the sensor body transmits or essentially transmits incoming signals. In other words, said sensor body exhibits a high transmittance. As a consequence, the sensor body when seen with a receiving device receiving the response signal such as an echographer will appear black to an observer. Upon an interaction of said sensor body with the parameter, in particular upon a spatial deformation of the sensor body which results in an elongation or compression of the sensor body, the transmission is modulated and an image of the sensor body will look brighter or darker, respectively. In the white sensor body configuration the combination of the components constituting the matrix and the substructures is chosen such that the sensor body reflects or essentially reflects incoming signals. In other words, said sensor body exhibits a low transmittance. As a consequence, the sensor body when seen with an echographer will appear white to the observer. Upon an interaction of said sensor body with the parameter, in particular upon a spatial deformation of the sensor body which results in an elongation of the sensor body, the transmission is modulated and an image of the sensor body will look darker or brighter, respectively. The third column of the table indicates the values of acoustic impedances of the matrix and the substructures, respectively.

| Matrix | Features | Acoustic impedance | Transmittance |
|---|---|---|---|
| **High Z** Pt | **Low Z** PDMS | 69.8 - 1.21 MRayl | 0.055 |
| **Low Z** PMMA | **High Z** Nitinol | 3.2 - 15.5 MRayl | 0.637 |
| **Low Z** Polyimide | **Low Z** SU-8 | 4.51- 3.4 MRayl | 0.963 |
| **High Z** Si | **High Z** Au | 19.6 - 63.8 MRayl | 0.805 |
| **Grey** PMMA | **High Z** Si | 3.2 - 19.6 MRayl | 0.540 |
| **Grey** Polyimide | **Low Z** PDMS | 4.51 -1.21 MRayl | 0.51 |
| **High Z** Ag | **Air** | 38 - 0.000429 MRayl | 0.000024 |
| **Low Z** Parylene-C | **Air** | 2.84 - 0.000429 MRayl | 0.00033 |

To this end it is conceivable that the sensor body is constituted by a single material having an ordered structure only. However, it is likewise conceivable that the sensor body comprises at least one further material having an ordered structure. The material having an ordered structure and the further material having an ordered structure can have different ordered structures. Materials of different ordered structures could be materials whose periodicity or aperiodicity is different. This could be achieved by providing a first matrix whose substructures are arranged according to a first arrangement and a second matrix whose substructures are arranged according to a second arrangement being different from the first arrangement, e.g. having substructures being further spaced apart from one another.

Said material having an ordered structure and the further material having an ordered structure could be arranged above one another with respect to a vertical direction and/or next to one another with respect to a horizontal direction running perpendicularly to the vertical direction and/or angled to one another.

For example, it is conceivable that the sensor body is formed of two or more materials having an ordered structure, such as two or more different metamaterials mentioned initially, and wherein said two or more materials having an ordered structure are layered on one another or are arranged immediately adjacent or laterally spaced from one another.

If two or more materials having an ordered structure are provided it is conceivable that one or more interfacing elements are provided at an interface between these materials having an ordered structure. An interfacing element is preferably provided by means of an acoustically transparent material. An acoustically transparent material is a material that is acoustically transparent with respect to its environment. Within the context of the present invention said environment corresponds to animal or human body tissue and be dependent on the particular sensor location. The acoustically transparent material preferably has a same or similar acoustic impedance as its environment and preferably exhibits a low absorption.

To this end various designs of the sensor body are conceivable. For example, the sensor body could have an essentially two-dimensional shape such as the shape of a membrane. However, other shapes such as a three-dimensional shape, e.g. a cube or a box or an ellipsoid or a sphere or a cylinder or a polyhedral etc., are likewise conceivable. Furthermore, the sensor body may be cut or shaped out of a material with the ordered structure so as to properly fit inside an implant.

The sensor body is preferably configured to adopt at least a first state and at least a second state, wherein the sensor body is configured to transform from its first state into its second state in response to the parameter. The sensor body, when in its first state, is preferably configured to yield at least one first response signal upon an interrogation of the sensor body with the interrogation signal. The sensor body, when in its second state, is preferably configured to yield at least one second response signal upon an interrogation of the sensor body with the interrogation signal. The at least one first response signal and/or the at least one second response signal is preferably associated with the one or more bulk effects of the at least one material having an ordered structure.

Thus, it is conceivable that the sensor body can adopt two or more states in response to the parameter. For example, the first state could correspond to a state where the sensor body is not subjected to the parameter, and the second state could correspond to a state where the sensor body is subjected to or has been subjected to the parameter. As mentioned initially, an interaction between the sensor body and the parameter could result in a deformation of the sensor body, for example an expansion of the sensor body. In this case the first state could be seen as the unexpanded state or the relaxed state of the sensor body and the second state could be seen as the expanded state of the sensor body.

In this context it should therefore be noted that the sensor body can exhibit different spatial extension when in the first state as compared to the second state. For example, a surface area of the sensor body when in the first state can be different such as smaller or larger than a surface area of the sensor body when in the second state. Additionally or alternatively a cross-section of the sensor body when in the first state can be different such as smaller or larger than a cross-section of the sensor body when in the second state.

Additionally or alternatively the sensor body is preferably configured such that a strain between the first state and the second state preferably is in the range of micro-$\varepsilon$ to milli-$\varepsilon$, e.g. in the range of 0.5 micro-$\varepsilon$ to 10 milli-$\varepsilon$, more preferably in the range of 0.5 micro-$\varepsilon$ to 1 milli-$\varepsilon$ or 0.5 micro-$\varepsilon$ to 2 milli-$\varepsilon$ or 0.5 micro-$\varepsilon$ to 3 milli-$\varepsilon$.

In these expressions the character $\varepsilon$ designates the strain as it is well known to the skilled person and commonly defined as:

$$\varepsilon \equiv \frac{\Delta l}{l_0} \quad (2)$$

Here, $\Delta l$ designates the amount by with the length of the sensor body changes and $l_0$ designates the original length of the sensor body. $\Delta l$ preferably is in the range of nanometer to 100 micrometer. $l_0$ preferably is about 100 to 2000000 times larger than $\Delta l$, preferably one to several cells sizes, i.e. one to several lengths (l) of one cell along one spatial direction as described in equation (2) above.

As mentioned above, the sensor body is preferably configured to yield at least one first response signal upon an interrogation of the sensor body with the interrogation signal when the sensor body is in its first state and is furthermore preferably configured to yield at least one second response signal upon an interrogation of the sensor body with the interrogation signal when the sensor body is in its second state. Said at least one first response signal and/or said at least one second response signal is preferably associated with the one or more bulk effects of the at least one material having an ordered structure as well.

Also in this case an open-loop operation and/or a closed-loop operation are conceivable. That is, it is conceivable that the parameter is determinable from a change between the first response signal and the second response signal.

Said change between the first response signal and the second response signal preferably corresponds to a spectral change of the first response signal and/or a spectral change of the second response signal, particularly preferably a change of the intensity of the first response signal and/or a change of the amplitude of the first response signal and/or a phase change of the first response signal and/or a modulation of the first response signal and/or a change of the intensity of the second response signal and/or a change of the amplitude of the second response signal and/or a phase change of the second response signal and/or a modulation of the second response signal.

However, it is likewise conceivable that said change between the first response signal and the second response signal corresponds to a spectral change between the first response signal and the second response signal, such as a difference between an intensity of the first response signal and an intensity of the second response signal and/or a difference between an amplitude of the first response signal and an amplitude of the second response signal and/or a difference between a phase of the first response signal and a phase of the second response signal and/or a difference between a modulation of the first response signal and a modulation of the second response signal.

When operated in the closed-loop it is conceivable that the parameter is determinable from a change of the interrogation signal that yields a particular first response signal and/or a particular second response signal. Here again it is preferred that said particular first response signal and/or said particular second response signal is a signal that is constant over time.

Furthermore, said change of the interrogation signal preferably is a change of the intensity of the interrogation signal and/or a change of the amplitude of the interrogation signal and/or a phase change of the interrogation signal and/or a modulation of the interrogation signal.

As mentioned previously, the sensor body can be configured to at least partially transmit the interrogation signal. Consequently, when the sensor body is in its first state, a first transmission spectrum constituting the first response signal can be generated and/or when the sensor body is in its second state, a second transmission spectrum constituting the second response signal can be generated. Additionally or alternatively, the sensor body can be configured to at least partially reflect the interrogation signal, whereby a first reflection spectrum constituting the first response signal can be generated when the sensor body is in its first state and/or a second reflection spectrum constituting the second response signal can generated when the sensor body is in its second state. Thus, the parameter can be determined by determining a change between the first transmission spectrum and the second transmission spectrum, the parameter being preferably determined by correlating and/or comparing said change with one or more table values and/or one or more calibration values. Additionally or alternatively the parameter can be determined by determining a change between the first reflection spectrum and the second reflection spectrum, the parameter being preferably determined by correlating and/or comparing said change with one or more table values and/or one or more calibration values.

It is of course conceivable that the sensor body can adapt two or more states, in particular a plurality of states, wherein the above explanations likewise apply to said plurality of states.

When at least a first state and a second state of the sensor body are investigated as just described the sensor can be said to perform a relative sensing.

However, it is likewise conceivable to investigate only one state of the sensor body, wherein the sensor can be said to perform an absolute sensing. For example, the above-mentioned second state of the sensor body, i.e. a state where the sensor body has been subjected to the parameter, could be investigated by irradiating the interrogation signal onto the sensor body in this second state and to determine the parameter from the response signal yielded by the sensor body in this second state.

Moreover, it should be noted that at least one first interrogation signal as well as at least one second interrogation signal could be used to interrogate the sensor body. These first and second interrogation signals preferably differ from one another, particularly preferably in one or more of their spectral properties such as their frequency and/or their intensity and/or their amplitude and/or their phase and/or their modulation. In this case it is furthermore preferred that the first interrogation signal is used to interrogate the sensor body when in its first state and that the second interrogation signal is used to interrogate the sensor body when in its second state.

Any explanations provided with respect to the response signal likewise apply to the first response signal and the second response signal and vice versa. Likewise, any explanations provided with respect to the interrogation signal likewise apply to the first interrogation signal and the second interrogation signal and vice versa. Etc. In other words, the components of the sensor and their abilities are the same when used in the absolute sensing and the relative sensing.

The implantable sensor can further comprise an encapsulation device that partly or entirely encapsulates the sensor body, said encapsulation device preferably being made of an acoustically transparent material and particularly preferably an acoustically transparent material having an ordered structure. The encapsulation device serves the purpose of protecting the sensor body towards an outside, e.g. from influences of the human or animal body when the sensor is implanted within a body.

The encapsulation device is preferably acoustically transparent with respect to the relevant local physiological environment the sensor is located in as well as with respect to a frequency or frequency band of the interrogation signal and a frequency or frequency band of the response signal. In other words, the encapsulation is preferably configured such that it does not interact with or perturb the interrogation signal and/or the response signal.

It is furthermore preferred that the encapsulation comprises or consists of a biocompatible material. The biocompatible material preferably comprises or consists of one or more polymers and/or copolymers therefor. Non-limiting examples of conceivable polymers are silicon-based polymers such as polymers comprising siloxane, e.g. Polysiloxane or Polydimethylsiloxane (PDMS), and/or thermoplastic polymers such as Poly(methyl methacrylate) (PMMA), and/or thermosetting polymers, and/or polymers of imide monomers, and/or thermosetting polyimides such as Polyimide, and/or epoxy-based polymers such as SU-8, and/or aromatic hydrocarbon polymers such as Polystyrene (PS), and/or polymers comprising a backbone comprising para-benzenediyl rings connected by 1, 2-ethanediyl bridges such as Parylene, in particular Parylene-C.

The implantable sensor can furthermore comprise at least one attachment device that allows or facilitates an attachment of the implantable sensor to hard tissue and/or soft tissue and/or an implant and/or on any other location of interest. The attachment device preferably corresponding to a mechanical attachment device such as suturing or clamps and/or a chemical attachment device such as an adhesive.

The implantable sensor is preferably configured for a wireless interrogation. A wireless interrogation is understood as utilizing acoustic waves instead of lines or cables or waveguides or for the purpose of transferring information and/or energy between a base station and the sensor.

The implantable sensor can be configured for an extracorporeal interrogation and/or an intracorporeal interrogation. The sensor being configured for an extracorporeal interrogation is understood as the capability of the sensor of being implantable within an animal or human body while it can be interrogated with an interrogation device that is arranged outside of said body.

The sensor being configured for an intracorporeal interrogation is understood as the capability of the sensor of being implantable within a body while it can be interrogated with an interrogation device that is also arranged within said body. For example, a transesophageal ultrasound device or intracardiac ultrasound device as known in the art could be used to interrogate the implantable sensor.

The parameter preferably is strain and/or stress and/or a pressure and/or a swelling and/or an absorption and/or a temperature and/or a biological process such as inflammation. It should be noted that the implantable sensor according to the invention enables a direct determination of the parameter of interest as well as an indirect determination of the parameter of interest. In the former case the sensor body is responsive to the parameter of interest itself. Consequently, the response signal will be associated with said parameter of interest and will allow a direct determination from the parameter of interest itself. In the latter case, the sensor body is responsive to a parameter that is associated with the parameter of interest, and wherein the parameter of interest can be deduced or derived from the parameter. In other words, the parameter is a quantity or the like that results in the parameter of interest or that generates the parameter of interest or that converts into the parameter of interest or that imposes the parameter of interest, etc. For example, the parameter that is determined from the response signal could be stress, and wherein the parameter of interest being strain is then determined from the stress.

It is particularly preferred that the implantable sensor is a strain sensor.

In a further aspect an implant comprising or consisting of an implantable sensor as described above is provided.

Any explanations that have been made with respect to the implantable sensor likewise apply to the implant comprising said implantable sensor and vice versa.

The implant preferably corresponds to an implant as it is known in the state of the art, i.e. a device that can be implanted within a human or animal body and which serves the purpose of sensing and transmitting physiological data of measured parameter or other derived parameters from it, or replacing a missing biological structure, supporting a damaged biological structure, or enhancing an existing biological structure. Non-limiting examples of such implants are sensory and neurological implants, cardiovascular implants, orthopedic implants, etc.

It is furthermore preferred that the implant corresponds to an implant that is commercially available in the state of the art.

The implantable sensor is preferably attached to or integrated into the implant, e.g. with the aid of one or more attachment devices mentioned above.

In a further aspect an assembly for determining at least one parameter, in particular a physical parameter, is provided. The assembly comprises at least one implantable sensor as described above. The assembly furthermore comprises at least one interrogation device that is configured to at least one interrogation signal comprising acoustic waves and at least one receiving device that is configured to receive the at least one response signal. In this regard it should be noted that the at least one interrogation device and the at least one receiving device can be provided by means of two separate and distinct components. However, it is likewise conceivable that the at least one interrogation device and the at least one receiving device are provided by means of a common device, which common device is configured to send one or more interrogation signals comprising acoustic waves as well as to receive the at least one response signal. In any case the assembly furthermore comprises at least one evaluation device that is configured to determine the at least one parameter from the at least one response signal and/or from the at least one interrogation signal.

It is particularly preferred that the assembly comprises standard medical equipment that is used for medical ultrasound, also called diagnostic sonography or ultrasonography. The advantages of providing an implantable sensor that can be interrogated with commercially medical equipment such as the medical ultrasound assembly described above are its simple, safe, widely accepted and inexpensive use. However, it should be noted that the implantable sensor is not limited to an interrogation with such standard medical equipment. To the contrary, it can be used with any other devices that are capable of sending interrogation signals comprising acoustic waves and receiving the corresponding response signals from the sensor body.

That is, the interrogation device is preferably configured to send one or more interrogation signals (or one or more first interrogation signals and/or one or more second interrogation signals) in the form of acoustic waves, in particular ultrasound waves. As mentioned earlier, a frequency of the interrogation signal preferably lies in the range of about 0.5 MHz to 30 MHz, more preferably in the range of about 1 MHz to 15 MHz. To this end it is conceivable that the interrogation signals are sent in the form of acoustic wave packets, i.e. as short bursts of acoustic waves. Thus, the interrogation device can be configured to operate in a pulsed mode. In this regard it is preferred when the interrogation device is configured to send pulsed interrogation signals having a pulse duration in the range of about 0.01 microsecond to 2 millisecond, preferably in the range of 0.05 microsecond to 1 millisecond,. Additionally or alternatively a number of cycles preferably lies between 1 and 5 cycles, more preferably between 2 and 4 cycles. However, it is likewise conceivable that the interrogation device is configured to operate in a continuous mode.

The interrogation device preferably comprises one or more transducers. A transducer preferably comprises 1 or more piezoelectric elements, preferably between 1 and 512 piezoelectric elements. Said piezoelectric elements preferably correspond to lead-zirconate-titanate (PZT) elements. If two or more transducers are provided, said transducers can be arranged in a linear array or in a matrix.

The receiving device is configured to receive one or more response signals (or one or more first response signals and/or one or more second response signals) from the sensor body. The receiving device can comprise one or more transducers that are configured to receive said response signals. The one or more transducers of the receiving device can be the same as or different from the one or more transducers of the interrogation device. That is, in the former case it is preferred that those transducers that send the interrogation signals are also used to detect the response signals. This case corresponds to the case of a regular sonography using a probe containing transducers that send the acoustic signals and which detected the reflected acoustic signals as an echo. In other words, the interrogation device and the receiving device can be provided by means of an echograph as it is known in the art. In the latter case it is likewise preferred that the one or more transducers of the receiving device correspond to the one or more transducers of the interrogation device, e.g. piezoelectric elements such as lead-zirconate-titanate (PZT) being arranged in an array or matrix, wherein the transducers of the receiving device and those of the interrogation device are arranged at different locations.

The evaluation device is preferably configured to determine the at least one parameter from the one or more response signals (or from the one or more first response signals and/or the one or more second response signals) when operated in the open-loop and/or from the one or more interrogation signals (or from the one or more first interrogation signals and/or from the one or more second interrogation signals) when operated in the closed-loop. As such, the evaluation device preferably is in operable connection with the receiving device and comprises dedicated equipment such a processor that allows a determination of the parameter from the response signals and/or from the interrogation signal. As said determination of the parameter is preferably achieved by comparing or correlating the response signals and/or the interrogation signals with one or more table values and/or calibration values, it is preferred that the evaluation device furthermore has access to or even comprises said table values and calibration values, respectively.

Calibration values could be obtained from a reference sensor that is used during a calibration step. Said calibration step could correspond to an initial calibration that is carried out prior to an implementation of the implantable sensor. It is likewise conceivable that the calibration step is a periodic recalibration.

In a further aspect a method of determining at least one parameter, in particular a physical parameter, using an implantable sensor as described above and/or an assembly as described above, wherein the method comprising the steps of i) sending at least one interrogation signal to the implantable sensor, ii) receiving the at least one response signal, and iii) determining the at least one parameter from the at least one response signal and/or from the at least one interrogation signal.

The steps i) to iii) are preferably performed in sequential order.

As mentioned earlier, when the sensor is operated in the open-loop operation it is preferred that the one or more interrogation signals (or the one or more first interrogation signals and/or the one or more second interrogation signals) are sent at a fixed frequency and/or at a fixed bandwidth. If the sensor is operated in the closed-loop operation it is preferred that the one or more interrogation signals (or the one or more first interrogation signals and/or the one or more second interrogation signals), in particular one or more frequencies of the one or more interrogation signals (or of the one or more first interrogation signals and/or of the one or more second interrogation signals), are continuously adjusted so as to maintain a particular response signal (or a particular one or more first response signals and/or a particular one or more second response signals).

It is furthermore preferred that the parameter is determined in real time. In other words, it is preferred that the implantable sensor corresponds to an analogic sensor and wherein any signal processing associated with digital electronics and the related architectures can be dispensed with. As a result, any delay in the data acquisition is reduced. However, it is of course likewise conceivable that the response signal(s) and/or the interrogation signal(s) are recorded and evaluated at a later point in time.

In a further aspect a method of producing an implantable sensor for determining at least one parameter, in particular a physical parameter, is provided. The method comprises the step of providing a sensor body. The sensor body is responsive to the at least one parameter. The sensor body is configured to yield at least one response signal upon an interrogation of the sensor body with at least one interrogation signal comprising acoustic waves. The at least one response signal is associated with the at least one parameter. The sensor body comprises or consists of at least one material having an ordered structure, and the at least one response signal is associated with one or more bulk effects of the at least one material having an ordered structure.

The implantable sensor particularly preferably corresponds to an implantable sensor as described above. As such, any explanations provided with regard to said implantable sensor or the assembly comprising said implantable sensor likewise apply for the method of producing the implantable sensor and vice versa.

Moreover, it is preferred that the sensor body is manufactured according to a subtractive micromachining technique such as etching or according to an additive micromachining such as a two photon polymerization, stereo lithography, soft material printing, selective laser sintering, direct metal laser sintering, and selective laser melting, etc. as they are known in the art. The two photon polymerization is a technology used to realize 3D structures in the range of nanometer to micrometer. It is based on a process where the absorption of two photons is conducted in a photosensitive material, the photoresist. The absorption of the laser pulse initiates the polymerization of the photoresist. After development, the polymerized material constitutes the final 3D shape of the sample.

In a further aspect a computer program product comprising computer program instructions that, when executed in a processor of the evaluation device of the assembly as described above cause the evaluation device to carry out the step of determining the at least one parameter from the at least one response signal and/or from the at least one interrogation signal.

To this end it is particularly preferred that the parameter is determined from the spectral content such as the intensity, the amplitude, the phase of the interrogation signal and/or of the response signal as explained previously.

BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,

| | |
|---|---|
| Fig. 1 | shows a schematical illustration of an interrogation of two implantable sensors with acoustic waves; |
| Fig. 2 | shows a perspective view of an implantable sensor that is arranged on an implant in a human heart; |
| Fig. 3a | shows a top view of the implant comprising the implantable sensor according to figure 2 in a first state; |
| Fig. 3b | shows a top view of the implant comprising the implantable sensor according to figure 2 in a second state; |
| Fig. 4 | shows a schematical illustration of an assembly comprising an implantable sensor being used for the determination of a parameter; |
| Fig. 5a | shows a schematical illustration of a sensor body of an implantable sensor on a microscale; |
| Fig. 5b-5e | show a schematical illustration of individual cells of the sensor body according to figure 5a; |
| Fig. 6 | shows a schematical illustration of a sensor body of an implantable sensor on a microscale; |
| Fig. 7 | shows a schematical illustration of a sensor body of an implantable sensor on a microscale; |
| Fig. 8a | shows a schematical illustration of a sensor body in a first state; |
| Fig. 8b | shows a schematical transmission spectrum yielded by the sensor body according to figure 8a; |
| Fig. 9a | shows a schematical illustration of the sensor body according to figure 8a in a second state; |
| Fig. 9b | shows schematical transmission spectra yielded by the sensor body in the first state and the second state; |
| Fig. 10 | shows the boundary conditions applied to a cell being utilized for the simulations depicted in figures 11 to 16d. |
| Fig. 11 | shows a simulation of the acoustic band structure of a periodic structure being constituted of cells according to figure 11 for different values of the strain applied to the cells; |
| Fig. 12 | shows a simulation of a transmission spectrum of a periodic structure being constituted of cells according to figure 11 for different values of strain applied to the cells and yielded upon an interrogation with a plane wave interrogation signal with direction (1,1); |
| Fig. 13a | shows a close-up of the transmission spectrum of figure 12 in the lower frequency range (0-10MHz) for a plane wave interrogation signal with direction (1,1); |
| Fig. 13b | shows a further close-up of the transmission spectrum of figure 13a in a small frequency range ~8.4 MHz; |
| Fig. 14a | shows a close-up of the transmission spectrum of figure 12 in the middle frequency range (13-18MHz) for a plane wave interrogation signal with direction (1,1); |
| Fig. 14b | shows a further close-up of the transmission spectrum of figure 14a in a small frequency range ~16.5MHz; |
| Fig. 15 | shows a close-up of the transmission spectrum of figure 12 in the middle frequency range (13-18MHz) for a plane wave interrogation signal with direction (1,0); |
| Fig. 16a | shows a simulation of the acoustic pressure field amplitude in space, where the interrogating signal is incident from the left and where the sensor body consists of 2x10 cells according to figure 11 at a strain of $\varepsilon = 0$; |
| Fig. 16b | shows a simulation of the acoustic pressure field amplitude in space, where the interrogating signal is incident from the left and where the sensor body consists of 2x10 cells according to figure 11 at a strain of $\varepsilon = 0.01$; |
| Fig. 16c | shows a simulation of the acoustic pressure field amplitude in space, where the interrogating signal is incident from the left and where the sensor body consists of 2x10 cells according to figure 11 at a strain of $\varepsilon = 0.03$; |
| Fig. 16d | shows a simulation of the acoustic pressure field amplitude in space, where the interrogating signal is incident from the left and where the sensor body consists of 2x10 cells according to figure 11 at a strain of $\varepsilon = 0.05$. |

DESCRIPTION OF PREFERRED EMBODIMENTS

With respect to the figures, different aspects of the implantable sensor 1 according to the invention will be discussed.

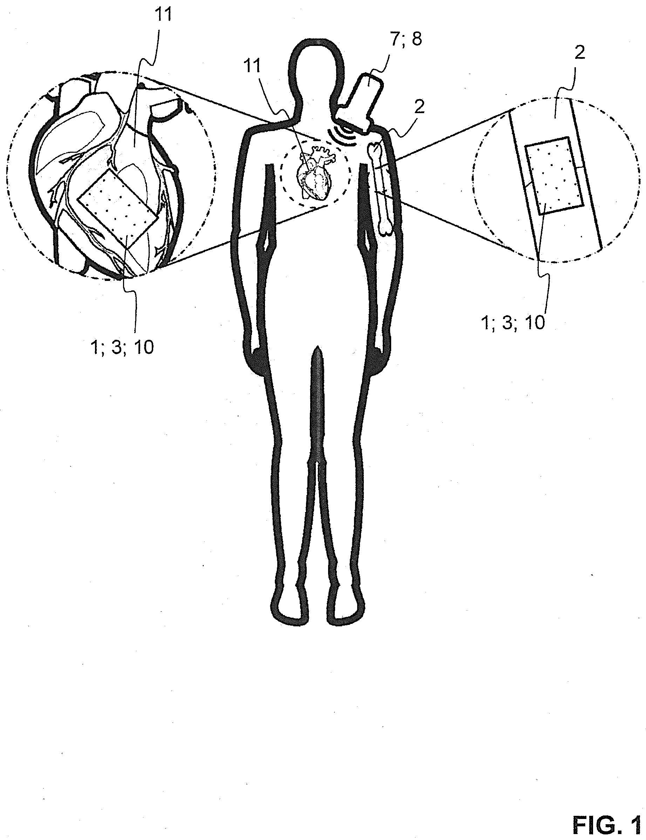

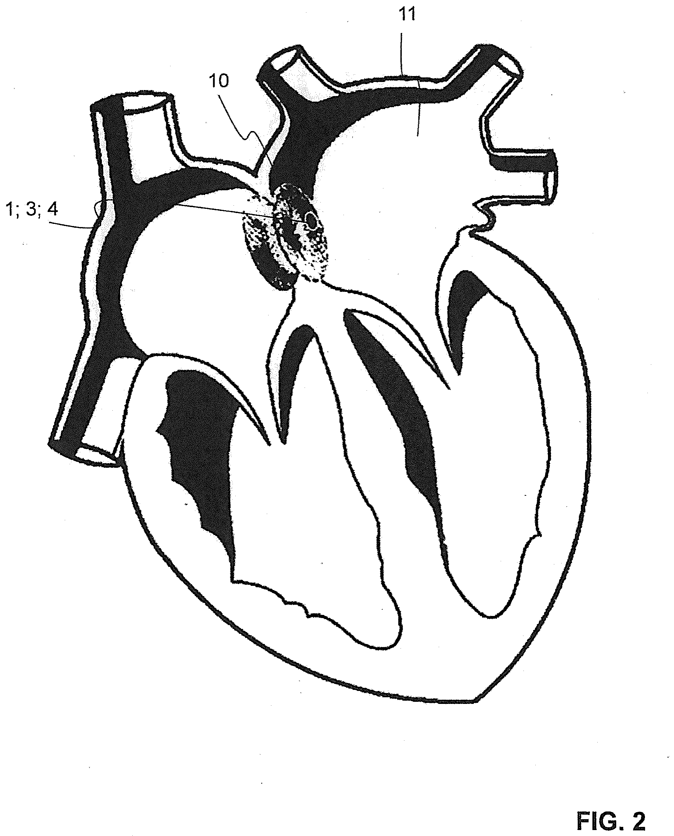

Figures 1 and 2 shall serve the purpose of explaining the overall design and working principle of the implantable sensor 1. More detailed explanations will be provided with respect to the further figures. In fact, figure 1 depicts two conceivable applications of the implantable sensor 1. Namely, on the one side an implantable sensor 1 is attached to a fractured bone 2 and serves the purpose of monitoring or assessing the bone healing. On the other side, another implantable sensor 1 is attached to the heart 11 and serves the purpose of monitoring or assessing cardiovascular events. In both cases the implantable sensor 1 is directly attached to the object 2, 11 to be monitored or assessed. These implantable sensors 1 can thus be seen as implants 10 themselves. In particular, in the former case the implantable sensor 1 can be said to constitute a cardiac implant 10 and in the latter case the implantable sensor 1 can be said to constitute an orthopedic implant 10. It is however likewise conceivable that the implantable sensor 1 is attached to a separate implant 10, see figures 2 to 3b. In particular, in figure 2 the sensor 1 is represented by the black circular area being surrounded by a white contour on the implant 10. In these figures the implant 10 corresponds to an occluder as it is known in the art, and wherein the implantable sensor 1 is attached to the occluder 10.

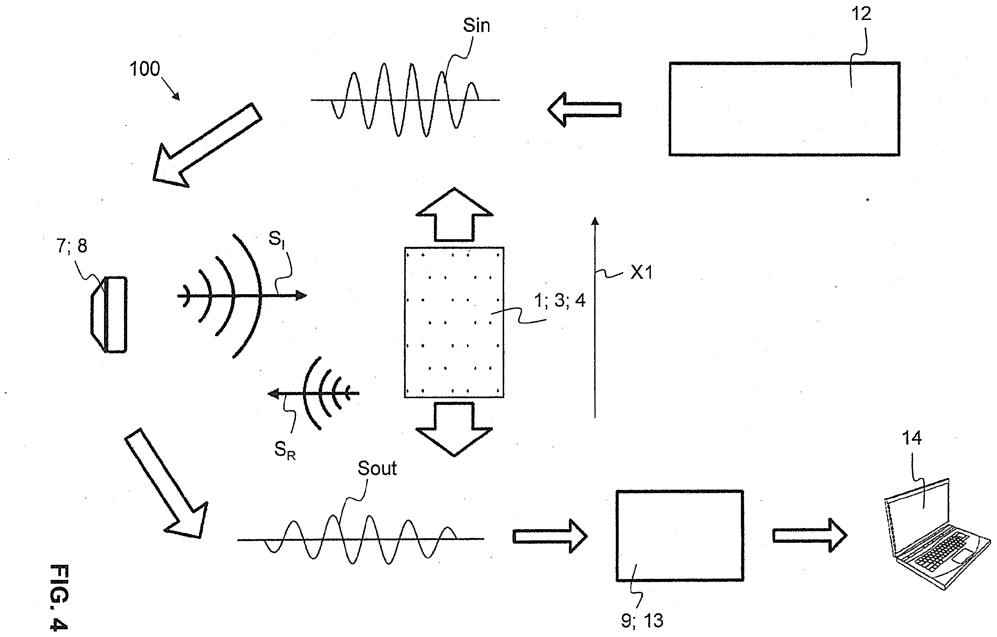

In any case, the implantable sensor 1 according to the invention comprises a sensor body 3 that is responsive to at least one parameter, in particular a physical parameter, and is configured to yield at least one response signal $S_R$ upon an interrogation of the sensor body 3 with at least one interrogation signal $S_I$ comprising acoustic waves. As schematically illustrated in figure 4, the interrogation signal $S_I$ can be provided by means of an interrogation device 7 such as a diagnostic sonography or ultrasonography used as standard medical equipment. As follows from figure 4 said interrogation device furthermore forms part of an assembly 100 that allows a determination of the parameter using the implantable sensor 1. Said assembly 100 may comprise an arbitrary waveform generator 12 which is used to generate an input signal Sin for the interrogation device 7. Said input signal is an electrical signal can have a particular waveform which is used to shape the interrogation signals $S_I$ in a desired way. A shaped interrogation signal $S_I$ is an interrogation signal $S_I$ having a particular amplitude or phase or frequency or duration or envelope or form, e.g. a sine waveform or a pulsed waveform, for example. It should be noted, however, that said waveform generator 12 is an entirely optional feature. In any case the assembly 100 furthermore comprises at least one receiving device 8 that is configured to receive the at least one response signal $S_R$ that is yielded by the sensor body 3 upon its interrogation with the acoustic waves of the interrogation signal $S_I$.

Upon receipt of the response signal $S_R$ the receiving device converts the ultrasound response signal $S_R$ into a corresponding electrical output signal $S_{out}$. It should be noted that the output signal $S_{out}$ is a faithful representation of $S_R$, and therefore it contains the same information about the parameter as the response signal $S_R$ yielded by the sensor body 3. In the following, reference is therefore still made to an evaluation or determination of the parameter from the response signal $S_R$, because $S_{out}$ is a mere electrical signal representation of the ultrasound response signal $S_R$. In particular, the response signal $S_R$ yielded by the sensor body 3 typically corresponds to a pressure wave response signal, and wherein said pressure wave response signal is converted by the receiving device 8 into an electrical output signal $S_{out}$ that can be processed by the evaluation device 9. The interrogation device 7 and the receiving device 8 can be provided by means of two separate components or two distinct components, respectively. In the scenario depicted in figures 1 and 4 the interrogation device 7 and the receiving device 8 are provided by means of the same component, in particular by a transducer, wherein said transducer emits the interrogation signal $S_I$ as well as receives the response signal $S_R$. However, it is likewise conceivable that a transducer for emitting the interrogation signal and a different transducer, which is optionally even located at a different location, is used for receiving the response are used instead. In any case the receiving device 8 is in communication with an evaluation device 9 that is configured to determine the at least one parameter from the response signal $S_R$, in particular from the output signal $S_{out}$ converted from $S_R$ by the receiving device 8. The evaluation device 9 comprises or corresponds to dedicated electronic equipment such as a processor 13 that allows the determination of the parameter from the response signal. Two ways of a determination of the parameter from the response signal are conceivable. Firstly, the parameter can be determined from the response signal $S_R$ received in an open-loop operation, wherein a change of the response signal $S_R$ is evaluated while the interrogation signal $S_I$ is kept constant. Secondly, the parameter can be determined from the response signal $S_R$ received in a closed-loop operation, wherein a change of the interrogation signal $S_I$ is evaluated while the response signal is kept constant. The evaluation of the change in the response signal $S_R$ as well as the evaluation of the change in the interrogation signal $S_I$ is performed by the evaluation device 9, in particular by its processor 13, and preferably includes a comparison or correlation or the like of the response signal $S_R$ or the interrogation signal $S_I$ with table values and/or calibration values. These table values or calibration values can be saved in a memory and locally on the evaluation device 9 or remotely. In the latter case the evaluation device 9 is in connection with a remote memory. The determined parameter can be outputted to an operator on an output device 14 such as a personal computer.

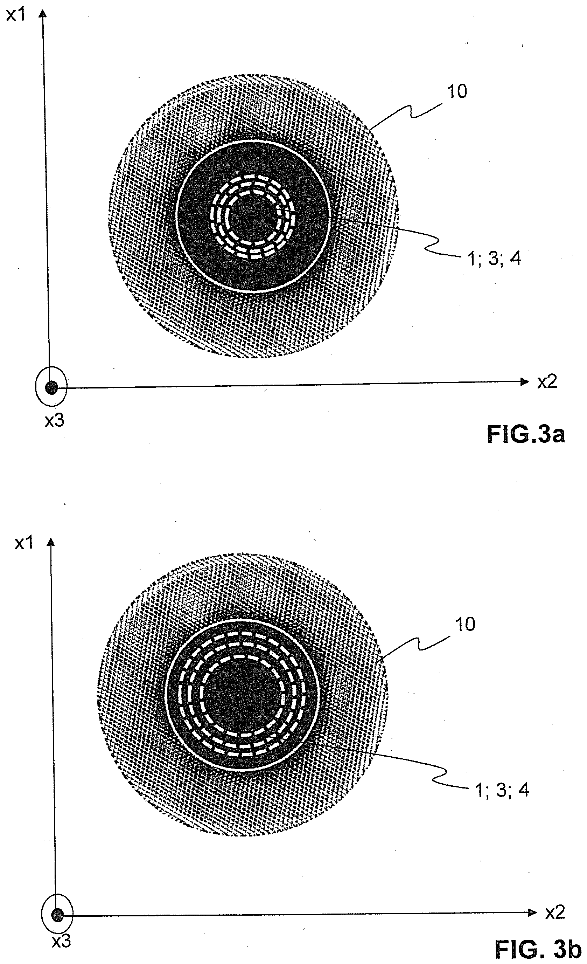

Various embodiments of the sensor body 3 are conceivable. In figure 1, the sensor body 3 is provided by means of an essentially rectangular membrane. In figures 2 to 3b, the sensor body 3 is likewise a membrane however of a circular shape. As is readily evident from a comparison of figure 3a with 3b, the sensor body 3 is configured to exhibit a deformation when subjected to the parameter. In the embodiment depicted in figures 3a and 3b, said deformation corresponds on the one side to a spatial deformation of the sensor body 3 along two spatial directions x1, x2 running perpendicular to one another. Since the sensor body 3 is of a circular shape said spatial deformation results in an enlargement and reduction of the radius defining the circular shape of the sensor body 3. In other words, the sensor body 3 is elongated and compressed upon an interaction with the parameter. This behavior is also recognizable from figure 4, wherein two arrows are used to symbolize an elongation of the rectangular sensor body along a spatial direction x1. On the other side the sensor body 3 is furthermore configured to deform along a third spatial direction x3 running perpendicularly to the first and second spatial directions x1, x2. In other words, the sensor body 3 can exhibit a vertical deflection along the third spatial direction x3, in particular along the -x3 direction and/or along the +x3 direction. In the depicted embodiment said vertical deflection is more pronounced than a horizontal deformation, i.e. a deformation along the first and second spatial directions x1, x2. In this case the cross-section of the sensor body essentially remains constant but an area defined by the sensor body is enlarged. Hence, the sensor body 3 can be said to comprise a buckled shape. The circles indicated by dashed lines in figures 3a and 3b shall illustrate this behavior.

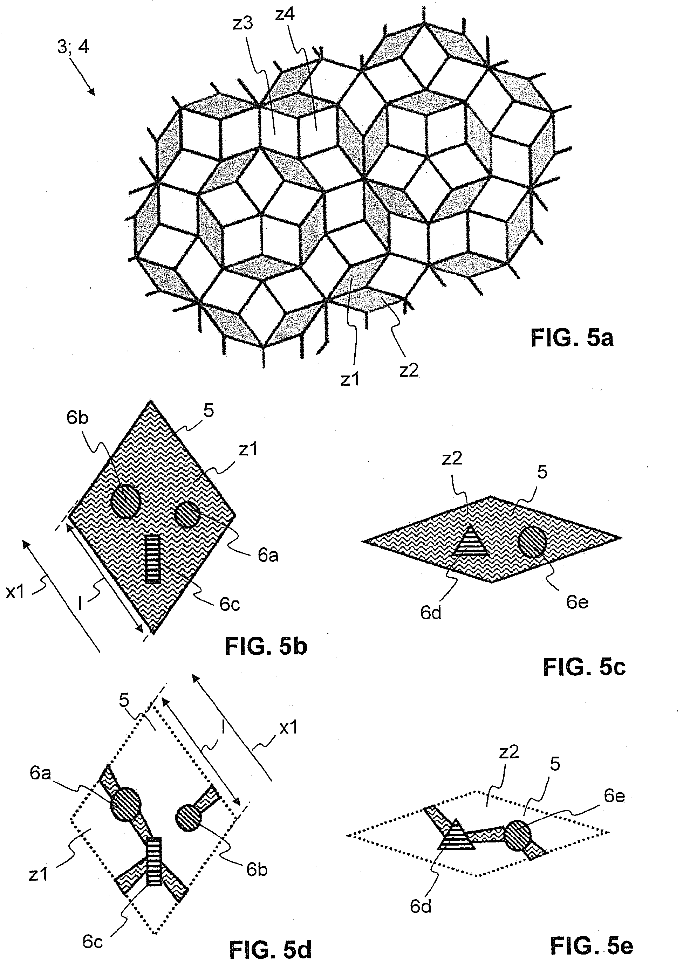

To this end the sensor body 3 comprises or consists of a material having an ordered structure 4, in particular a material having a lattice or a lattice-like structure. Said lattice or lattice-like structure is defined by means of a plurality of cells z1-z4 see figures 5a to 7. A cell z1, z2, etc. is understood as the smallest repeating unit of the material having an ordered structure 4.

Figures 5a to 5e depict a first embodiment of a sensor body 3. Said sensor body 3 is constituted by a plurality of cells z1 of a first cell type and a plurality of cells z2 of a second cell type being different from the cells z1 of the first cell type. In fact, the cells z1 of the first cell type are depicted in figures 5b and 5d and are provided by means of a first matrix 5, within which first substructures 6a-6c are arranged. The first substructures 6a-6c are of a different shape, namely circular substructures and rectangular substructures. it should be noted that any other arbitrary shape is likewise conceivable. Figures 5c and 5e depict the cells z2 of the second cell type, which comprises second substructures 6d-6e having a different shape and arrangement than the substructures 6a-6c of the first cell type depicted in figures 5b and 5d . Said second substructures 6a-6e are arranged within a matrix 5 as well. Whereas the substructures 6a-6c of the first cell type z1 depicted in figure 5b and the substructures 6d-6e of the second cell type z2 depicted in figure 5c are arranged within a matrix that is continuous, the substructures 6a-6c of the first cell type z1 depicted in figure 5d and the substructures 6d-6e of the second cell type z2 depicted in figure 5e are comprised within a matrix that is shaped into solid connectors.

In each case the cell dimensions of the cells z1, z2 are chosen such that a length *l* of one cell z1, z2 with respect to a spatial direction x1 is in the order of magnitude of the speed of sound c within the material having an ordered structure constituting said one cell divided by a frequency f of the interrogation signal or a frequency of the response signal. This approximation is furthermore based on the assumption that the length l of said cell z1, z2 is in the same order of magnitude as an integer number n of (1/2) of a wavelength $\lambda$ of the interrogation signal $S_I$ and/or of the response signal $S_R$, such that the following relationship can be used to determine the length *l* of said cell: $l = \frac{n\lambda}{2} = n\,\frac{c}{2f}$ .

Because an interrogation of the sensor body 3 is performed with an interrogation signal $S_I$ comprising acoustic waves it is preferred that the length *l* of one cell z1, z2 along one spatial direction x1 is in the range of 1 micrometer to 10 millimeter. Such a length *l* enables an interrogation with acoustic waves having a frequency in the range of about 0.5 MHz to 30 MHz.

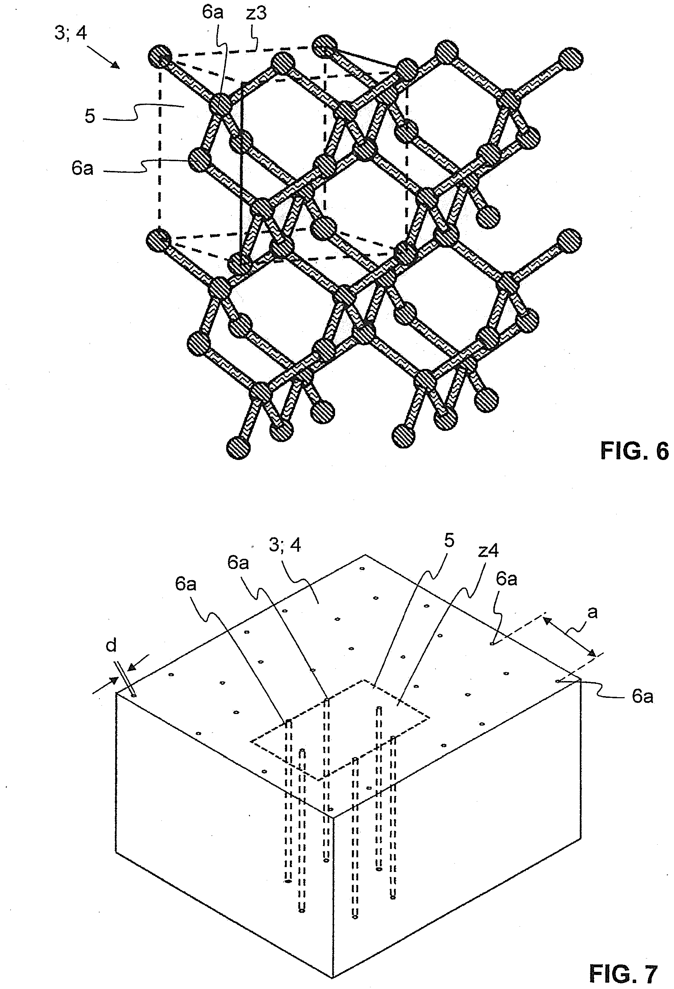

Figures 6 and 7 depict further conceivable embodiments of the sensor body 3, i.e. of the material having an ordered structure 4 constituting the sensor body 3. That is, figure 6 is a further example of a three dimensional periodically structured material 4. The sensor body 3 according to this embodiment is constituted by one type of cells z3 only. Said periodically structured material 4 again comprises a matrix 5 corresponding to solid connectors as discussed with reference to figures 5d and 5e and comprising substructure 6a. Figure 7 discloses an example of a two-dimensional periodically structured material 4, wherein said structured material 4 is again constituted by one type of cells z4 only. A particular cell z4 of said structured material 4 is composed of a homogeneous matrix 5 with cylindrical substructures 6a arranged therein. The sensor body, which is constituted by a plurality of such cells, has here the shape of a cuboid. As indicated in figure 7, a distance (a) among the substructures 6a is preferably about 0.5 micrometer or larger. A cross-section d of an individual substructure 6a preferably is about 0.1 micrometer or larger.

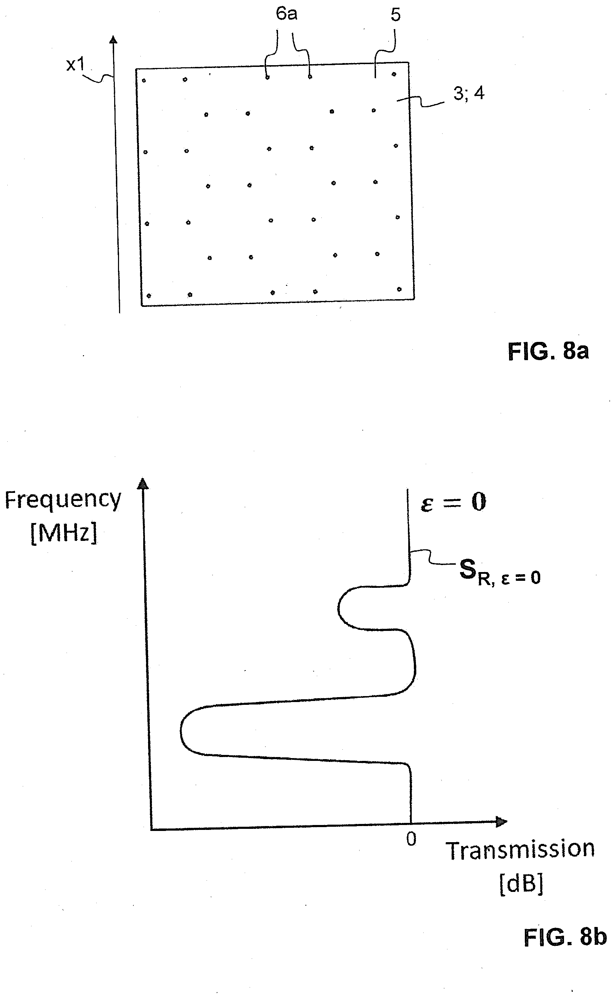

All of these sensor bodies 3 have in common that an interaction of the sensor body 3 with the parameter results in a deformation of the sensor body 3, wherein said deformation can express itself in a distortion of the lattice or the lattice-like structure. When the lattice or the lattice-like structure is distorted, one or more lattice constants of the lattice or the lattice-like structure are changed accordingly. In other words, the lattice constants are configured to exhibit a change when the sensor body is subjected to the parameter. However, it is likewise conceivable that the sensor body 3 comprises or consists of a material having an ordered structure 4 whose lattice or lattice-like structure does not distort upon an interaction with the parameter. Instead, it is conceivable that the material having the ordered structure 4 constituting the sensor body 3 comprises an acoustic band structure and/or an acoustic band gap which changes upon an interaction of the sensor body 3 with the parameter. It is furthermore conceivable that the material having an ordered structure 4 exhibits one or more acoustic properties that change upon an interaction of the sensor body 3 with the parameter. A change of the acoustic properties such as an acoustic impedance, a Young's modulus or the density of the material having an ordered structure can have an impact on the acoustic band structure such that their change influences the acoustic band structure. However, even if these acoustic properties remain constant the acoustic band structure can still be affected by a deformation such as a simple geometrical distortion, e.g. a spatial deformation. Moreover, one or more of these changes result in a change of the reflectance and/or transmittance of the material having an ordered structure 4. Said changes in the reflectance or in the transmittance can be observed by changes in the response signal $S_R$ corresponding to a reflection spectrum or in the response signal $S_R$ corresponding to a transmission spectrum or in the interrogation signal $S_I$ resulting in a particular response signal $S_R$ in the form of a particular reflection spectrum or in the interrogation signal $S_I$ resulting in a particular response signal $S_R$ in the form of a particular transmission spectrum, as will be explained now with reference to figures 8a to 9b.

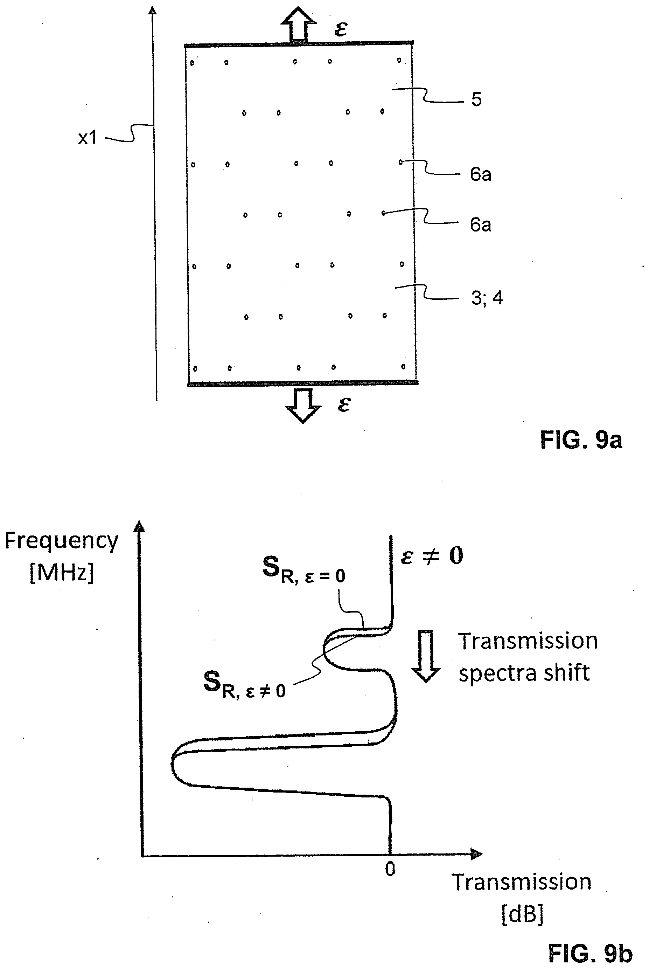

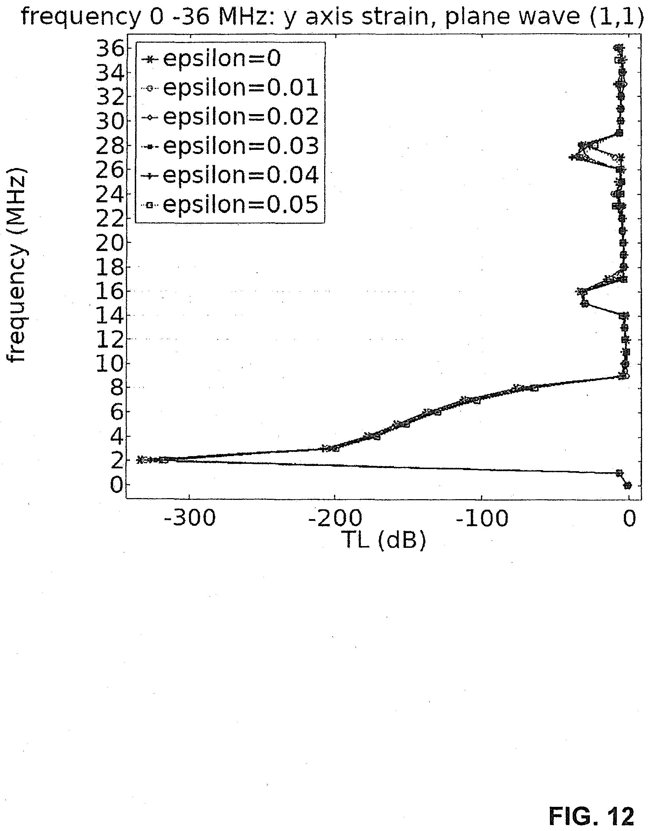

Namely, in these figures an external sensor interrogation in the low MHz ultrasound range shall be illustrated, wherein an external incident ultrasound wave is generated and used to directly interrogate the material as explained with reference to figures 1 and 4. One or several properties of the sensor body such as its acoustic band structure or its acoustic impedance are modulated, i.e. changed, by the parameters to be measured (e.g. strain or any other physical or chemical quantity resulting in strain). Once a pressure is applied in/within an organ or tissue of the human body, the stress configuration of the sensor body changes accordingly, and its lattice structure or lattice-like structure is affected. The consequent change of the lattice constants, when a strain is applied, impacts the reflective and/or transmissive acoustic properties of the sensor body. In summary, the values of transmission and reflection of the incident ultrasound wave in the sensor body changes in real-time with the applied strain. Both operational methods are conceivable, i.e. (1) open-loop, wherein changes in transmission/reflection of the ultrasound waves or wave packets at a fixed frequency or narrow-band are measured, and (2) closed-loop, wherein a frequency of the ultrasound transceiver is continuously adjusted to maintain a given transmission/reflection value. As a specific example, the determination of the parameter being strain can be illustrated by means of a deformation of a rectangular sensor body 3 along a spatial direction x1 and the recording of transmission spectra as response signals $S_R$. Since the implantable sensor 1 is here used for the determination of strain the implantable sensor 1 can be seen as a strain sensor. It should be noted that an analogous situation applies if the response signal $S_R$ is recorded as reflection spectrum or if the parameter is determined by analyzing the interrogation signals $S_I$, i.e. during a closed-loop operation. In figures 8a the sensor body 3 is depicted in a relaxed state, i.e. in a state where no interaction of the sensor body 3 with the parameter is taking place. Consequently, no strain acts on the sensor body 3 such that the strain $\varepsilon$ is zero. An interrogation of the sensor body 3 with acoustic waves at a given ultrasound excitation range results in a particular transmission response spectrum $S_{R,\,\varepsilon=0}$ which is depicted in figure 8b. The application of a strain $\varepsilon \neq 0$ results here in the mentioned deformation of the sensor body 3 along the spatial direction x1. The sensor body 3 can be said to be in a deformed state. Because of a change in the acoustic band structure the transmission spectrum yielded by the sensor body as response signal $S_{R,\,\varepsilon\neq0}$ in the deformed state is changed as compared to the transmission spectrum yielded by the sensor body as response signal $S_{R,\,\varepsilon=0}$ in the relaxed state, see figure 9b. In the present situation the interaction of the sensor body with the parameter results in a frequency shift of the transmission spectrum. Here, said frequency shift corresponds to a change in the position on the frequency axis of the transition between high-transmission (0 dB) and low-transmission (<0 dB), see also figure 12.

However, the interaction can manifest itself also in further spectral changes, such as a change of the intensity or of the amplitude or of the phase of the response signal when investigated in the open-loop operation or of the interrogation signal when investigated in the closed-loop operation. An analysis of any one of these changes enables the determination of the parameter.

If only one state of the sensor body 3 is investigated so as to determine the parameter the implantable sensor 1 is used for an absolute sensing. However, it is likewise conceivable that the implantable sensor 1 is used for a relative sensing. In this latter case at least two states of the sensor body 1 are investigated, and wherein a change between these two states upon an interaction of the sensor body 3 with the parameter is investigated. For example, the relaxed state of the sensor body 3 depicted in figure 8a could be defined as a first state and the expanded state of the sensor body 3 depicted in figure 9a could be defined as a second state. The sensor body 3, when in its first state, can be said to yield a first response signal upon an interrogation of the sensor body 3 with a first interrogation signal. The sensor body 3, when in its second state, can be said to yield a second response signal upon an interrogation of the sensor body 3 with a second interrogation signal. Hence, in this relative sensing the parameter can be determined by investigating a change between the first and second response signals and/or a change between the first and second interrogation signals depending on the mode of operation of the sensor body (closed-loop operation or open-loop operation).

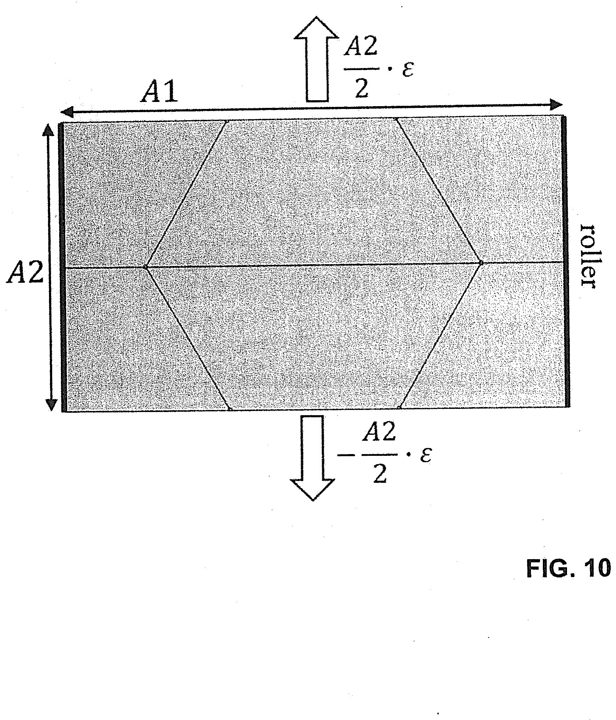

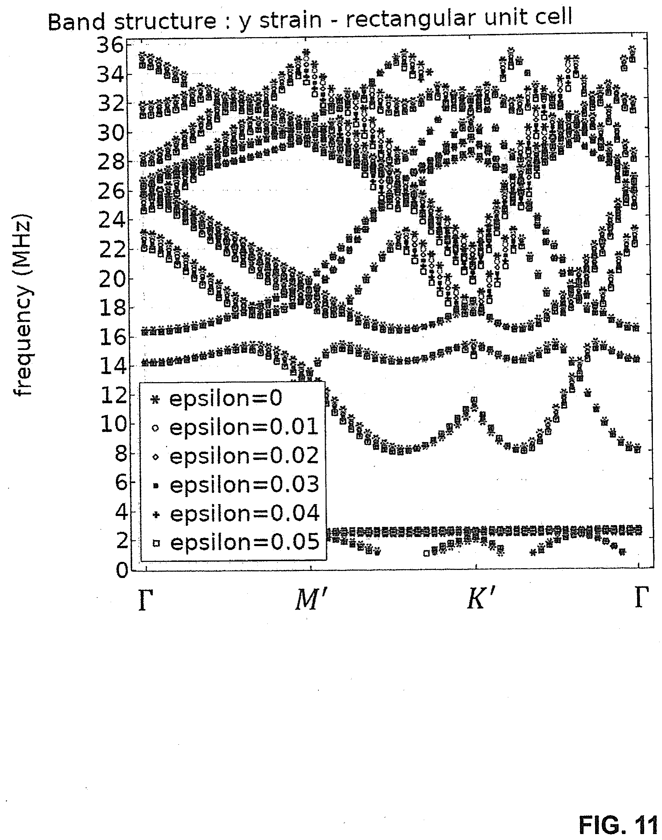

With respect to figures 10 to 16d further characteristics of the implantable sensor 1 according to the invention will be discussed by means of FEM simulation case studies. To this end figure 10 depicts the boundary conditions being applied to a cell of a sensor body 3, i.e. of the material having an ordered structure 4. Said cell corresponds here to the cell type z4 as depicted in figure 7 and it is utilized for the simulations depicted in figures 11 to 16d. A longitudinal strain is applied along a width of the cell and on both sides of the cell, wherein said sides are free to deform (roller boundary condition on FEM simulation). In other words, left and right are sliding / roller boundary conditions are applied, wherein strain is applied parallel to the direction of A2. Here said strain is a multiple of ($\pm$A2/2) and varies between 1% $\div$ 5% with a step width of 1 %.

Figure 11 depicts the acoustic band structure (dispersion relations) of the periodic structure being constituted with the cell depicted in figure 10 for different values of strain applied, i.e. a strain $\varepsilon$ of 0, 0.01, 0.02, 0.03, 0.04 and 0.05. The x-axis is referred to the reciprocal space and crystallographic directions. From this simulation the downward shift of the bands, accordingly to different values of strain applied, can be observed.

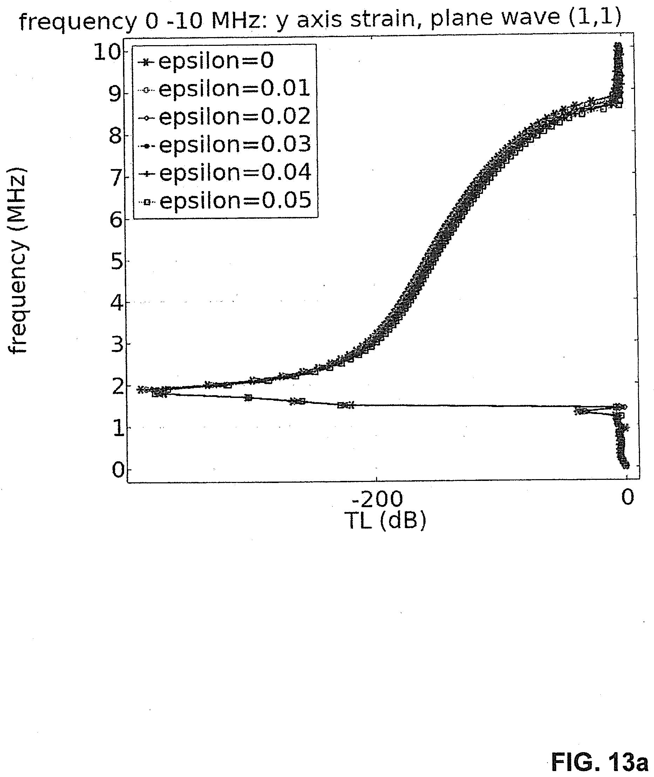

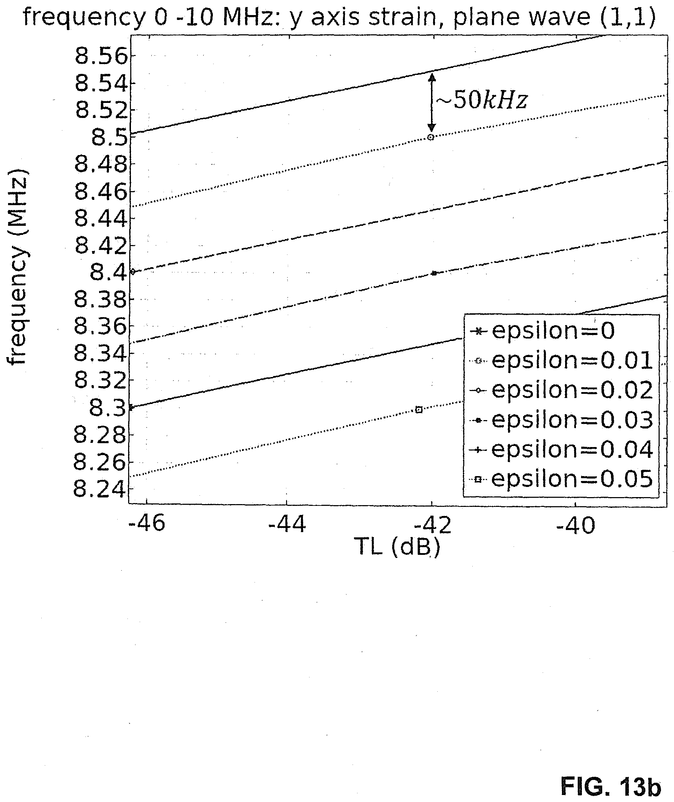

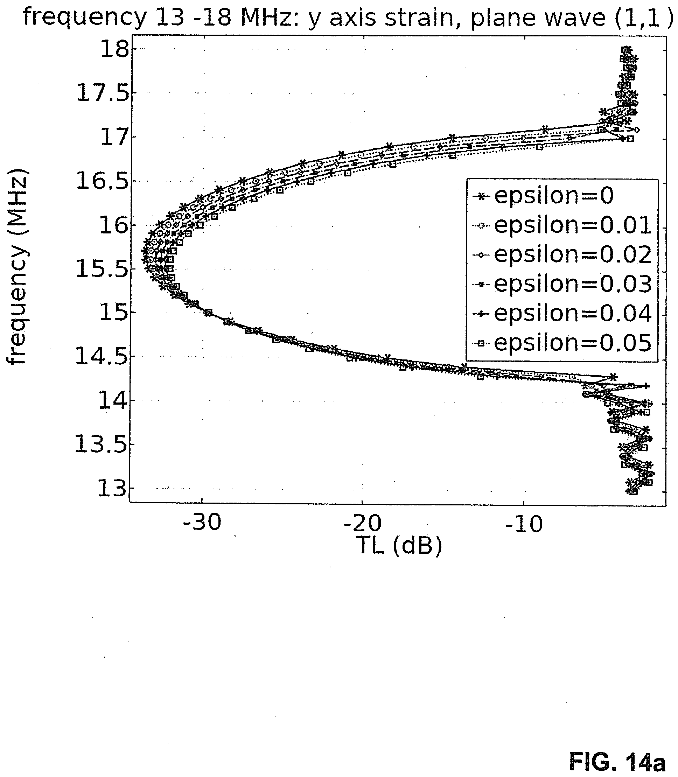

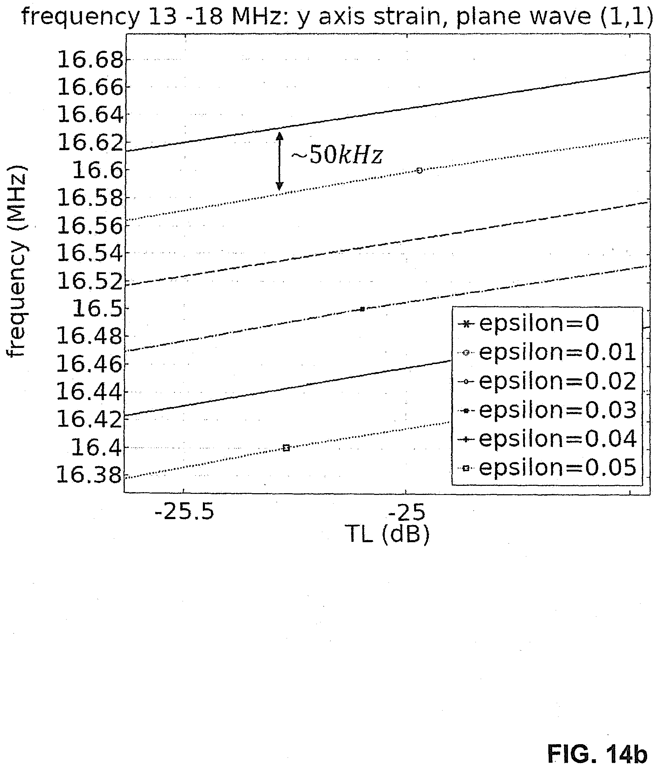

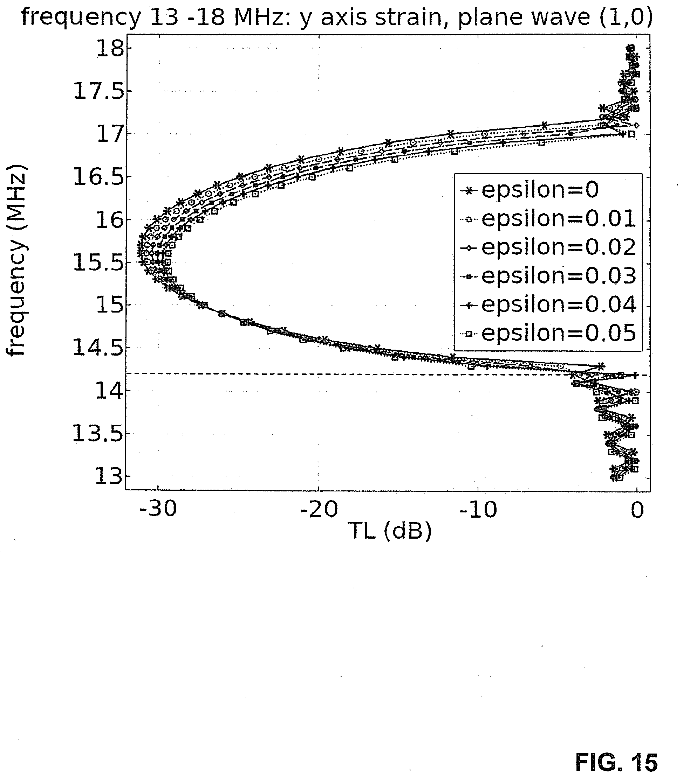

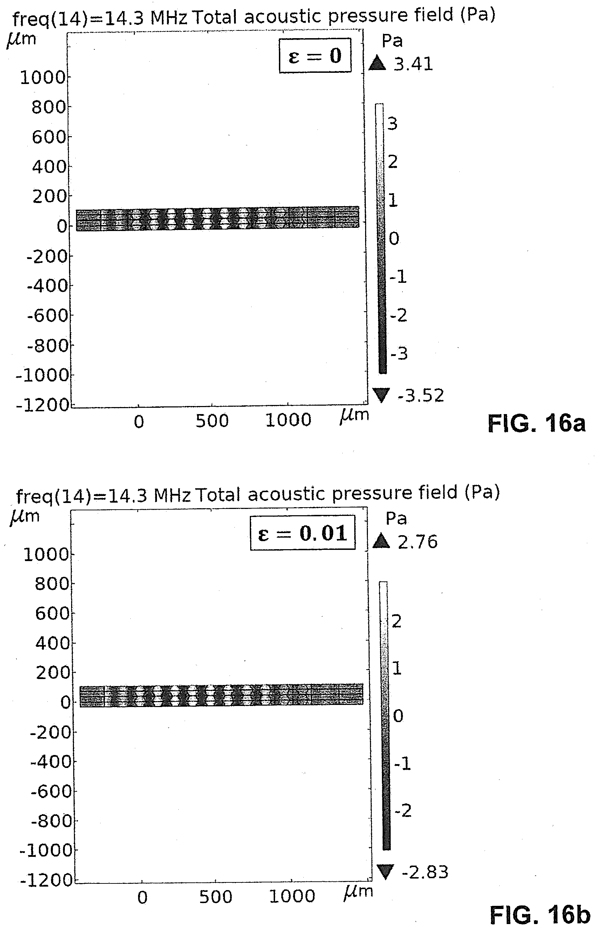

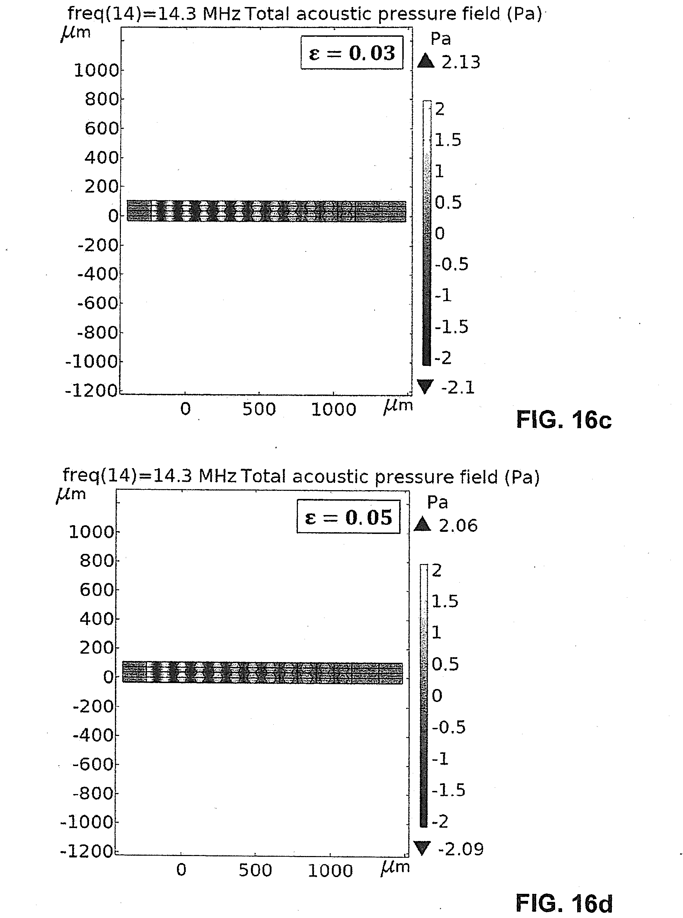

Figure 12 depicts the transmission spectrum of the periodic structure with the cell according to figure 11 for different values of the applied strain (again $\varepsilon$ = 0 to 0.05) for a plane wave interrogation signal with direction (1,1). The x-axis of the transmission spectrum is expressed in function of the Transmission Loss (TL): $10 \cdot log \frac{P_{out}}{P_{in}}$ [dB] . Figure 13a depicts a close-up of the transmission spectrum depicted in figure 12 in the lower frequency range of 0-10 MHz for a plane wave interrogation signal with direction (1,1). Figure 13b shows a further close-up of the transmission spectrum in Fig 13a in a small frequency range of ~8.4 MHz. Figure 14a shows a close-up of the transmission spectrum in Fig. 12 in the middle frequency range of 13-18MHz for a plane wave interrogation signal with direction (1,1). Figure 14b shows a further close up of the transmission spectrum in Fig 13a in a small frequency range of ~16.5MHz. From these simulations it follows that the frequency shift corresponding to a strain variation of 1% is equivalent to 50 *kHz*. Figure 15 shows a close-up of the transmission spectrum of figure 12 in the middle frequency range of 13-18MHz for a plane wave interrogation signal with direction (1,0). The dashed line represents the equivalent frequency of the excitation signal that gives rise to the simulated acoustic pressure field, in fig. 16a-16d

Figures 16a to 16d depict simulations of the acoustic pressure field amplitude in space, where the interrogation signal parallel to direction (1,0) and where the sensor body consists of 2x10 cells according to figure 10 for different values of strain. As follows from these figures an increased value of strain modulates the percentage of signal transmitted and reflected in the sensor body, i.e. the material having an ordered structure, with respect to the transmission spectra. The modulation of the reflected properties of the sensor body with respect to the strain value applied is visible through the scale color bar.

## LIST OF REFERENCE SIGNS

| | | | |
|---|---|---|---|
| 1 | implantable sensor | 14 | output device |
| 2 | bone | | |
| 3 | sensor body | $S_R$ | response signal |
| 4 | material having ordered structure | $S_I$ | interrogation signal |
| | | $S_{in}$ | input signal |
| 5 | matrix | $S_{out}$ | output signal |
| 6a, 6b, ... | substructure | x1, x2,... | spatial direction |
| 7 | interrogation device | a | distance |
| 8 | receiving device | d | cross-section |
| 9 | evaluation device | z1, z2, ... | cell |
| 10 | implant | l | length |
| 100 | assembly | c | speed of sound |
| 11 | heart | f | frequency |
| 12 | arbitrary waveform generator | | |
| 13 | processor | | |

**Claims**

1. An implantable sensor (1) for determining at least one parameter, in particular a physical parameter,

   wherein the implantable sensor (1) comprises a sensor body (3),
   wherein the sensor body (3) is responsive to the at least one parameter,
   wherein the sensor body is configured to yield at least one response signal ($S_R$) upon an interrogation of the sensor body (3) with at least one interrogation signal ($S_I$) comprising acoustic waves,
   wherein the at least one response signal ($S_R$) is associated with the at least one parameter,

**characterized in that** the sensor body (3) comprises or consists of at least one material having an ordered structure (4), and

**in that** the at least one response signal ($S_R$) is associated with one or more bulk effects of the at least one material having an ordered structure (4),

wherein the material having an ordered structure (4) comprises an acoustic band structure and/or an acoustic band gap, and wherein said acoustic band structure and/or acoustic band gap is configured to exhibit a change when the sensor body (3) is subjected to the parameter, and wherein the change of the acoustic band structure and/or of the acoustic band gap correspondingly changes the response signal ($S_R$), and/or

wherein the material having an ordered structure (4) comprises a lattice and/or a lattice-like structure, and wherein said lattice and/or lattice-like structure is distorted when the sensor body (3) is subjected to the parameter, and wherein the distortion of the lattice and/or the distortion of the lattice-like structure correspondingly changes the response signal ($S_R$).

2. The implantable sensor (1) according to claim 1, wherein the material having an ordered structure (4) is configured to exhibit a deformation when subjected to the parameter, and/or
wherein the material having an ordered structure (4) exhibits one or more acoustic properties, and wherein said one or more acoustic properties are configured to change when the sensor body (3) is subjected to the parameter.

3. The implantable sensor (1) according to claim 2, wherein the deformation is a one-dimensional or a multi-dimensional spatial deformation along one or more spatial directions (x1, x2, x3) and/or a shear deformation, and/or
wherein the acoustic properties are at least one of an acoustic impedance (4), at least one mechanical property (4) such as the Young's modulus of the material (4), a density of the material (4), an acoustic band structure of the material having an ordered structure (4), or an acoustic band gap of the material having an ordered structure (4), a change in the acoustic band structure and/or in the band gap particularly preferably resulting in a change of the reflectance of the material having an ordered structure (4) and/or in a change of the transmittance of the material having an ordered structure (4).

4. The implantable sensor (1) according to any one of the preceding claims, wherein the at least one parameter is determinable from a change in the response signal ($S_R$), said change preferably being a spectral change, particularly preferably a change of the intensity of the response signal ($S_R$) and/or a change of the amplitude of the response signal ($S_R$) and/or a phase change of the response signal ($S_R$) and/or a modulation of the response signal ($S_R$), and/or wherein the at least one parameter is determinable from a change of the interrogation signal ($S_I$) that yields a particular response signal ($S_R$), said particular response signal ($S_R$) preferably being a signal that is constant over time.

5. The implantable sensor (1) according to any one of the preceding claims, wherein the material having an ordered structure (4) comprises a lattice and/or a lattice-like structure that is constituted by one or more cells ($z1$, $z2$, ...), and wherein a length ($l$) of one cell ($z1$, $t2$, ...) with respect to a spatial direction ($x1$) is in the order of magnitude of the speed of sound ($c$) within the material having an ordered structure (4) divided by a frequency ($f$) of the interrogation signal ($S_I$) or a frequency of the response signal ($S_R$), and/or
wherein a length ($l$) of one cell ($z1$, $z2$, ...) with respect to a spatial direction ($x1$) is in the range of 1 micrometer to 10 millimeter, preferably in the range of 10 micrometer to 1 millimeter, and/or at least 1 micrometer, preferably at least 10 micrometer, and/or up to 10 millimeter, preferably up to 1 millimeter.

6. The implantable sensor (1) according to any one of the preceding claims, wherein the material having an ordered structure (4) comprises at least one matrix (5) and one or more substructures (6a, 6b, ...) being arranged within the at least one matrix (5), and

wherein an acoustic impedance of the matrix (5) preferably differs from the acoustic impedance of the substructure (6a, 6b, ...), and/or
wherein a distance ($a$) among the substructures (6a, 6b, ...) is preferably about 0.5 micrometer or larger, preferably in the range of about 0.5 micrometer to 9 millimeter, more preferably in the range of about 1 micrometer and 5 millimeter and/or
wherein a cross-section ($d$) of an individual substructure (6a, 6b, ...) preferably is about 0.1 micrometer or larger, preferably in the range of about 1 micrometer to 100 micrometer,

7. The implantable sensor (1) according to any one of the preceding claims, wherein the sensor body (3) is configured to adopt at least a first state and at least a second state,

wherein the sensor body (3) is configured to transform from its first state into its second state in response to the parameter,

wherein the sensor body (3), when in its first state, is configured to yield at least one first response signal ($S_R$) upon an interrogation of the sensor body (3) with the interrogation signal ($S_I$),

wherein the sensor body (3), when in its second state, is configured to yield at least one second response signal ($S_R$) upon an interrogation of the sensor body (3) with the interrogation signal ($S_I$), and wherein the at least one first response signal ($S_R$) and/or the at least one second response signal ($S_R$) is associated with the one or more bulk effects of the at least one material having an ordered structure (4).

8. The implantable sensor (1) according to any one of the preceding claims, further comprising an encapsulation device that partly or entirely encapsulates the sensor body (3), said encapsulation device preferably being made of an acoustically transparent material and particularly preferably an acoustically transparent material having an ordered structure (4), and/or

wherein the implantable sensor (1) further comprises at least one attachment device that allows or facilitates an attachment of the implantable sensor (1) to hard tissue and/or soft tissue and/or an implant, the attachment device preferably corresponding to a mechanical attachment device such as suturing or clamps and/or a chemical attachment device such as an adhesive.

9. The implantable sensor (1) according to any one of the preceding claims, wherein the implantable sensor (1) is configured for a wireless interrogation, and/or

wherein the implantable sensor (1) is configured for an extracorporeal interrogation and/or an intracorporeal interrogation.

10. The implantable sensor (1) according to any one of the preceding claims, wherein the parameter is strain and/or stress and/or a pressure and/or a swelling and/or an absorption and/or a temperature and/or a biological process such as inflammation, and/or

wherein the implantable sensor (1) is a strain sensor.

11. An implant (10) comprising or consisting of an implantable sensor (1) according to any one of the preceding claims.

12. An assembly (100) for determining at least one parameter, in particular a physical parameter, the assembly comprising:

- at least one implantable sensor (1) as claimed in any one of claims 1-10;
- at least one interrogation device (7) that is configured to send at least one interrogation signal ($S_I$) comprising acoustic waves;
- at least one receiving device (8) that is configured to receive the at least one response signal ($S_R$); and
- at least one evaluation device (9) that is configured to determine the at least one parameter from the at least one response signal ($S_R$) and/or from the at least one interrogation signal ($S_I$).

13. A method of determining at least one parameter, in particular a physical parameter, using an implantable sensor (1) according to any one of claims 1-10 and/or an assembly (100) according to claim 12, the method comprising the steps of:

- sending at least one interrogation signal ($S_I$) to the implantable sensor (1);
- receiving the at least one response signal ($S_R$); and
- determining the at least one parameter from the at least one response signal ($S_R$) and/or from the at least one interrogation signal ($S_I$).

14. A method of producing an implantable sensor (1) for determining at least one parameter, in particular a physical parameter,

wherein the method comprises the step of providing a sensor body (3),

wherein the sensor body (3) is responsive to the at least one parameter,

wherein the sensor body (3) is configured to yield at least one response signal ($S_R$) upon an interrogation of the sensor body (3) with at least one interrogation signal ($S_I$) comprising acoustic waves,

wherein the at least one response signal ($S_R$) is associated with the at least one parameter,

**characterized in that** the sensor body (3) comprises or consists of at least one material having an ordered structure (4), and

**in that** the at least one response signal ($S_R$) is associated with one or more bulk effects of the at least one material having an ordered structure (4),

wherein the material having an ordered structure (4) comprises an acoustic band structure and/or an acoustic band gap, and wherein said acoustic band structure and/or acoustic band gap is configured to exhibit a change when the sensor body (3) is subjected to the parameter, and wherein the change of the acoustic band structure and/or of the acoustic band gap correspondingly changes the response signal ($S_R$), and/or

wherein the material having an ordered structure (4) comprises a lattice and/or a lattice-like structure, and wherein said lattice and/or lattice-like structure is distorted when the sensor body (3) is subjected to the parameter, and wherein the distortion of the lattice and/or the distortion of the lattice-like structure correspondingly changes the response signal ($S_R$).

15. A computer program product comprising computer program instructions that, when executed in a processor of the evaluation device (9) of the assembly (100) according to claim 12 cause the evaluation device (9) to carry out the step of determining the at least one parameter from the at least one response signal ($S_R$) and/or from the at least one interrogation signal ($S_I$).

**Patentansprüche**

1. Implantierbarer Sensor (1) zur Bestimmung mindestens eines Parameters, insbesondere eines physikalischen Parameters,

wobei der implantierbare Sensor (1) einen Sensorkörper (3) umfasst,

wobei der Sensorkörper (3) auf den mindestens einen Parameter anspricht,

wobei der Sensorkörper derart ausgebildet ist, dass er bei einer Abfrage des Sensorkörpers (3) mit mindestens einem Abfragesignal ($S_I$), das akustische Wellen umfasst, mindestens ein Antwortsignal ($S_R$) liefert,

wobei das mindestens eine Antwortsignal ($S_R$) mit dem mindestens einen Parameter assoziiert ist,

**dadurch gekennzeichnet, dass** der Sensorkörper (3) mindestens ein Material mit einer geordneten Struktur (4) umfasst oder daraus besteht, und

dass das mindestens eine Antwortsignal ($S_R$) mit einem oder mehreren Volumeneffekten des mindestens einen Materials mit der geordneten Struktur (4) assoziiert ist,

wobei das Material mit der geordneten Struktur (4) eine akustische Bandstruktur und/oder eine akustische Bandlücke umfasst, und wobei die akustische Bandstruktur und/oder die akustische Bandlücke derart ausgebildet ist, dass sie eine Änderung aufweist, wenn der Sensorkörper (3) dem Parameter ausgesetzt ist, und wobei die Änderung der akustischen Bandstruktur und/oder der akustischen Bandlücke das Antwortsignal ($S_R$) entsprechend ändert, und/oder

wobei das Material mit der geordneten Struktur (4) ein Gitter und/oder eine gitterähnliche Struktur umfasst, und wobei das Gitter und/oder die gitterähnliche Struktur verzerrt wird, wenn der Sensorkörper (3) dem Parameter ausgesetzt wird, und wobei die Verzerrung des Gitters und/oder die Verzerrung der gitterähnlichen Struktur das Antwortsignal ($S_R$) entsprechend ändert.

2. Implantierbarer Sensor (1) gemäss Anspruch 1, wobei das Material mit einer geordneten Struktur (4) derart ausgebildet ist, dass es eine Verformung aufweist, wenn es dem Parameter ausgesetzt wird, und/oder

wobei das Material mit der geordneten Struktur (4) eine oder mehrere akustische Eigenschaften aufweist, und wobei die eine oder mehreren akustischen Eigenschaften derart ausgebildet sind, dass sie sich ändern, wenn der Sensorkörper (3) dem Parameter ausgesetzt wird.

3. Implantierbarer Sensor (1) gemäss Anspruch 2, wobei die Verformung eine eindimensionale oder eine mehrdimensionale räumliche Verformung entlang einer oder mehrerer räumlicher Richtungen (x1, x2, x3) und/oder eine Scherverformung ist, und/oder

wobei die akustischen Eigenschaften mindestens eine der folgenden Eigenschaften umfassen: eine akustische Impedanz (4), mindestens eine mechanische Eigenschaft (4) wie beispielsweise das Elastizitätsmodul des Materials (4), eine Dichte des Materials (4), eine akustische Bandstruktur des Materials mit der geordneten Struktur (4) oder eine akustische Bandlücke des Materials mit der geordneten Struktur (4), wobei eine Änderung der akustischen Bandstruktur und/oder der Bandlücke insbesondere bevorzugt zu einer Änderung des Reflexionsvermögens des Materials mit der geordneten Struktur (4) und/oder zu einer Änderung des Transmissionsvermögens des Materials mit der geordneten Struktur (4) führt.

**4.** Implantierbarer Sensor (1) gemäss einem der vorstehenden Ansprüche, wobei der mindestens eine Parameter aus einer Änderung des Antwortsignals $(S_R)$ bestimmbar ist, wobei die Änderung vorzugsweise eine spektrale Änderung, insbesondere vorzugsweise eine Änderung der Intensität des Antwortsignals $(S_R)$ und/oder eine Änderung der Amplitude des Antwortsignals $(S_R)$ und/oder eine Phasenänderung des Antwortsignals $(S_R)$ und/oder eine Modulation des Antwortsignals $(S_R)$ ist und/oder

wobei der mindestens eine Parameter aus einer Änderung des Abfragesignals $(S_I)$ bestimmbar ist, das ein bestimmtes Antwortsignal $(S_R)$ ergibt, wobei das bestimmte Antwortsignal $(S_R)$ vorzugsweise ein Signal ist, das über die Zeit konstant ist.

**5.** Implantierbarer Sensor (1) gemäss einem der vorstehenden Ansprüche, wobei das Material mit der geordneten Struktur (4) ein Gitter und/oder eine gitterartige Struktur umfasst, die aus einer oder mehreren Zellen (z1, z2, ...) besteht, und wobei eine Länge (I) einer Zelle (z1, t2, ...) in Bezug auf eine räumliche Richtung (x1) in der Grössenordnung der Schallgeschwindigkeit (c) innerhalb des Materials mit einer geordneten Struktur (4) geteilt durch eine Frequenz (f) des Abfragesignals $(S_I)$ oder eine Frequenz des Antwortsignals $(S_R)$ liegt, und/oder

wobei eine Länge (I) einer Zelle (z1, z2, ...) in Bezug auf eine räumliche Richtung (x1) im Bereich von 1 Mikrometer bis 10 Millimeter, vorzugsweise im Bereich von 10 Mikrometern bis 1 Millimeter, und/oder mindestens 1 Mikrometer, vorzugsweise mindestens 10 Mikrometer, und/oder bis zu 10 Millimeter, vorzugsweise bis zu 1 Millimeter ist.

**6.** Implantierbarer Sensor (1) gemäss einem der vorstehenden Ansprüche, wobei das Material mit der geordneten Struktur (4) mindestens eine Matrix (5) und eine oder mehrere Unterstrukturen (6a, 6b, ...) umfasst, die innerhalb der mindestens einen Matrix (5) angeordnet sind, und

wobei sich die akustische Impedanz der Matrix (5) vorzugsweise von der akustischen Impedanz der Unterstruktur (6a, 6b, ...) unterscheidet, und/oder

wobei ein Abstand (a) zwischen den Unterstrukturen (6a, 6b, ...) vorzugsweise etwa 0,5 Mikrometer oder grösser ist, vorzugsweise im Bereich von etwa 0.5 Mikrometer bis 9 Millimeter, noch bevorzugter im Bereich von etwa 1 Mikrometer und 5 Millimeter, und/oder

wobei ein Querschnitt (d) einer einzelnen Unterstruktur (6a, 6b, ...) vorzugsweise etwa 0.1 Mikrometer oder grösser ist, vorzugsweise im Bereich von etwa 1 Mikrometer bis 100 Mikrometer.

**7.** Implantierbarer Sensor (1) gemäss einem der vorstehenden Ansprüche, wobei der Sensorkörper (3) derart ausgebildet ist, dass er mindestens einen ersten Zustand und mindestens einen zweiten Zustand annimmt,

wobei der Sensorkörper (3) derart ausgebildet ist, dass er sich in Reaktion auf den Parameter von seinem ersten Zustand in seinen zweiten Zustand umwandelt,

wobei der Sensorkörper (3), wenn er sich in seinem ersten Zustand befindet, derart ausgebildet ist, dass er bei einer Abfrage des Sensorkörpers (3) mit dem Abfragesignal $(S_I)$ mindestens ein erstes Antwortsignal $(S_R)$ erzeugt,

wobei der Sensorkörper (3), wenn er sich in seinem zweiten Zustand befindet, derart ausgebildet ist, dass er bei einer Abfrage des Sensorkörpers (3) mit dem Abfragesignal $(S_I)$ mindestens ein erstes Antwortsignal $(S_R)$ erzeugt, und wobei das mindestens eine erste Antwortsignal $(S_{(R)})$ und/oder das mindestens eine zweite Antwortsignal $(S_R)$ mit dem einen oder den mehreren Volumeneffekten des mindestens einen Materials mit der geordneten Struktur (4) assoziiert ist.

**8.** Implantierbarer Sensor (1) gemäss einem der vorstehenden Ansprüche, weiter umfassend eine Kapselungsvorrichtung, die den Sensorkörper (3) teilweise oder vollständig umschliesst, wobei die Kapselungsvorrichtung vorzugsweise aus einem akustisch transparenten Material und besonders bevorzugt aus einem akustisch transparenten Material mit einer geordneten Struktur (4) besteht, und/oder

wobei der implantierbare Sensor (1) weiter mindestens eine Befestigungsvorrichtung umfasst, die eine Befestigung des implantierbaren Sensors (1) an Hartgewebe und/oder Weichgewebe und/oder einem Implantat ermöglicht oder erleichtert, wobei die Befestigungsvorrichtung vorzugsweise einer mechanischen Befestigungsvorrichtung wie einer Naht oder Klammern und/oder einer chemischen Befestigungsvorrichtung wie einem Klebstoff entspricht.

**9.** Implantierbarer Sensor (1) gemäss einem der vorstehenden Ansprüche, wobei der implantierbare Sensor (1) für eine drahtlose Abfrage ausgebildet ist, und/oder

wobei der implantierbare Sensor (1) für eine extrakorporale Abfrage und/oder eine intrakorporale Abfrage ausgebildet ist.

10. Implantierbarer Sensor (1) gemäss einem der vorstehenden Ansprüche, wobei der Parameter eine Dehnung und/oder eine Spannung und/oder ein Druck und/oder eine Schwellung und/oder eine Absorption und/oder eine Temperatur und/oder ein biologischer Prozess wie eine Entzündung ist, und/oder
wobei der implantierbare Sensor (1) ein Dehnungssensor ist.

11. Implantat (10), das einen implantierbaren Sensor (1) gemäss einem der vorstehenden Ansprüche umfasst oder aus diesem besteht.

12. Anordnung (100) zum Bestimmen mindestens eines Parameters, insbesondere eines physikalischen Parameters, wobei die Anordnung umfasst:

   - mindestens einen implantierbaren Sensor (1) gemäss einem der Ansprüche 1 bis 10;
   - mindestens eine Abfragevorrichtung (7), die derart ausgebildet ist, dass sie mindestens ein Abfragesignal ($S_I$) sendet, das akustische Wellen umfasst;
   - mindestens eine Empfangsvorrichtung (8), die derart ausgebildet ist, dass sie mindestens ein Antwortsignal ($S_R$) empfängt; und
   - mindestens eine Auswertevorrichtung (9), die derart ausgebildet ist, dass sie den mindestens einen Parameter aus dem mindestens einen Antwortsignal ($S_R$) und/oder aus dem mindestens einen Abfragesignal ($S_I$) bestimmt.

13. Verfahren zum Bestimmen mindestens eines Parameters, insbesondere eines physikalischen Parameters, unter Verwendung eines implantierbaren Sensors (1) gemäss einem der Ansprüche 1 bis 10 und/oder einer Anordnung (100) gemäss Anspruch 12, wobei das Verfahren die folgenden Schritte umfasst:

   - Senden mindestens eines Abfragesignals ($S_I$) an den implantierbaren Sensor (1);
   - Empfangen des mindestens einen Antwortsignals ($S_R$); und
   - Bestimmen des mindestens einen Parameters aus dem mindestens einen Antwortsignal ($S_R$) und/oder aus dem mindestens einen Abfragesignal ($S_I$).

14. Verfahren zur Herstellung eines implantierbaren Sensors (1) zur Bestimmung mindestens eines Parameters, insbesondere eines physikalischen Parameters,

   wobei das Verfahren den Schritt des Bereitstellens eines Sensorkörpers (3) umfasst,
   wobei der Sensorkörper (3) auf den mindestens einen Parameter anspricht,
   wobei der Sensorkörper (3) derart ausgebildet ist, dass er bei einer Abfrage des Sensorkörpers (3) mit mindestens einem Abfragesignal ($S_I$), das akustische Wellen umfasst, mindestens ein Antwortsignal ($S_R$) liefert,
   wobei das mindestens eine Antwortsignal ($S_R$) mit dem mindestens einen Parameter assoziiert ist,
   **dadurch gekennzeichnet, dass** der Sensorkörper (3) mindestens ein Material mit einer geordneten Struktur (4) umfasst oder daraus besteht, und
   dass das mindestens eine Antwortsignal ($S_R$) mit einem oder mehreren Volumeneffekten des mindestens einen Materials mit der geordneten Struktur (4) assoziiert ist,
   wobei das Material mit der geordneten Struktur (4) eine akustische Bandstruktur und/oder eine akustische Bandlücke umfasst, und wobei die akustische Bandstruktur und/oder die akustische Bandlücke derart ausgebildet ist, dass sie eine Änderung aufweist, wenn der Sensorkörper (3) dem Parameter ausgesetzt ist, und wobei die Änderung der akustischen Bandstruktur und/oder der akustischen Bandlücke das Antwortsignal ($S_R$) entsprechend ändert, und/oder
   wobei das Material mit der geordneten Struktur (4) ein Gitter und/oder eine gitterähnliche Struktur umfasst, und wobei das Gitter und/oder die gitterähnliche Struktur verzerrt wird, wenn der Sensorkörper (3) dem Parameter ausgesetzt wird, und wobei die Verzerrung des Gitters und/oder die Verzerrung der gitterähnlichen Struktur das Antwortsignal ($S_R$) entsprechend ändert.

15. Ein Computerprogrammprodukt, das Computerprogrammbefehle umfasst, die, wenn sie in einem Prozessor der Auswertevorrichtung (9) der Anordnung (100) gemäss Anspruch 12 ausgeführt werden, die Auswertevorrichtung (9) veranlassen, den Schritt des Bestimmens des mindestens einen Parameters aus dem mindestens einen Antwortsignal ($S_R$) und/oder aus dem mindestens einen Abfragesignal ($S_I$) auszuführen.

**Revendications**

1. Capteur implantable (1) permettant de déterminer au moins un paramètre, notamment un paramètre physique,

   dans lequel le capteur implantable (1) comprend un corps de capteur (3),
   dans lequel le corps de capteur (3) est sensible à l'au moins un paramètre,
   dans lequel le corps de capteur est configuré pour produire au moins un signal de réponse (S$_R$) lors d'une interrogation du corps de capteur (3) avec au moins un signal d'interrogation (S$_I$) comprenant des ondes acoustiques,
   dans lequel l'au moins un signal de réponse (S$_R$) est associé à l'au moins un paramètre,
   **caractérisé en ce que** le corps de capteur (3) comprend ou est constitué d'au moins un matériau ayant une structure ordonnée (4), et
   **en ce que** l'au moins un signal de réponse (S$_R$) est associé à un ou plusieurs effets de volume de l'au moins un matériau ayant une structure ordonnée (4),
   dans lequel le matériau ayant une structure ordonnée (4) comprend une structure de bande acoustique et/ou une bande interdite acoustique , et dans lequel ladite structure de bande acoustique et/ou de bande interdite acoustique est configurée pour présenter un changement lorsque le corps de capteur (3) est soumis au paramètre, et dans lequel le changement de la structure de bande acoustique et/ou de bande interdite acoustique change le signal de réponse (S$_R$) de manière correspondante,
   et/ou dans lequel le matériau ayant une structure ordonnée (4) comprend un réseau et/ou une structure de type réseau, et dans lequel ladite structure et/ou structure de type réseau est déformée lorsque le corps de capteur (3) est soumis au paramètre, et dans lequel la déformation du réseau et/ou de la structure de type réseau change le signal de réponse (S$_R$) de manière correspondante.

2. Le capteur implantable (1) selon la revendication 1, dans lequel le matériau ayant une structure ordonnée (4) est configuré pour présenter une déformation lorsqu'il est soumis au paramètre, et/ou
   dans lequel le matériau ayant une structure ordonnée (4) présente une ou plusieurs propriétés acoustiques, et dans lequel lesdites une ou plusieurs propriétés acoustiques sont configurées pour changer lorsque le corps de capteur (3) est soumis au paramètre.

3. Le capteur implantable (1) selon la revendication 2, dans lequel la déformation est une déformation spatiale unidimensionnelle ou multidimensionnelle le long d'une ou plusieurs directions spatiales (x1, x2, x3) et/ou une déformation de cisaillement, et/ou
   dans lequel les propriétés acoustiques sont au moins une parmi une impédance acoustique (4), au moins une propriété mécanique (4) telle que le module de Young du matériau (4), une densité du matériau (4), une structure de bande acoustique du matériau ayant une structure ordonnée (4), ou une bande interdite acoustique du matériau ayant une structure ordonnée (4), un changement de la structure de bande acoustique et/ou de la bande interdite entraînant de préférence un changement de la réflectance du matériau ayant une structure ordonnée (4) et/ou un changement de la transmittance du matériau ayant une structure ordonnée (4).

4. Le capteur implantable (1) selon l'une quelconque des revendications précédentes, dans lequel l'au moins un paramètre peut être déterminé à partir d'un changement du signal de réponse (S$_R$), ledit changement étant de préférence un changement spectral, de manière particulièrement préférée un changement de l'intensité du signal de réponse (S$_R$) et/ou une variation de l'amplitude du signal de réponse (S$_R$) et/ou une variation de phase du signal de réponse (S$_R$) et/ou une modulation du signal de réponse (S$_R$), et/ou
   dans lequel l'au moins un paramètre peut être déterminé à partir d'un changement du signal d'interrogation (S$_I$) qui produit un signal de réponse particulier (S$_R$), ledit signal de réponse particulier (S$_R$) étant de préférence un signal qui est constant dans le temps.

5. Le capteur implantable (1) selon l'une quelconque des revendications précédentes, dans lequel le matériau ayant une structure ordonnée (4) comprend un réseau et/ou une structure de type réseau constituée d'une ou plusieurs cellules (z1, z2, ...), et dans lequel une longueur (l) d'une cellule (z1, t2, ...) par rapport à une direction spatiale (x1) est de l'ordre de grandeur de la vitesse du son (c) dans le matériau ayant une structure ordonnée (4) divisée par une fréquence (f) du signal d'interrogation (S$_I$) ou une fréquence du signal de réponse (S$_R$), et/ou
   dans lequel la longueur (l) d'une cellule (z1, z2, ...) par rapport à une direction spatiale (x1) est comprise entre 1 micromètre et 10 millimètres, de préférence entre 10 micromètres et 1 millimètre, et/ou d'au moins 1 micromètre, de préférence d'au moins 10 micromètres, et/ou jusqu'à 10 millimètres, de préférence jusqu'à 1 millimètre.

6. Le capteur implantable (1) selon l'une quelconque des revendications précédentes, dans lequel le matériau ayant une structure ordonnée (4) comprend au moins une matrice (5) et une ou plusieurs sous-structures (6a, 6b, ...) disposées à l'intérieur de l'au moins une matrice (5), et

dans lequel une impédance acoustique de la matrice (5) diffère de préférence de l'impédance acoustique de la sous-structure (6a, 6b, ...), et/ou
dans laquelle une distance (a) entre les sous-structures (6a, 6b, ...) est de préférence d'environ 0,5 micromètre ou plus, de préférence dans la plage d'environ 0,5 micromètre à 9 millimètres, plus préférablement dans la plage d'environ 1 micromètre à 5 millimètres et/ou
dans laquelle une section transversale (d) d'une sous-structure individuelle (6a, 6b, ...) est de préférence d'environ 0,1 micromètre ou plus, de préférence dans la plage d'environ 1 micromètre à 100 micromètres.

7. Le capteur implantable (1) selon l'une quelconque des revendications précédentes, dans lequel le corps de capteur (3) est configuré pour adopter au moins un premier état et au moins un deuxième état,

dans lequel le corps de capteur (3) est configuré pour passer de son premier état à son second état en réponse au paramètre,
dans lequel le corps de capteur (3), lorsqu'il est dans son premier état, est configuré pour produire au moins un premier signal de réponse ($S_R$) lors d'une interrogation du corps de capteur (3) avec le signal d'interrogation ($S_I$),
dans lequel le corps de capteur (3), lorsqu'il est dans son deuxième état, est configuré pour produire au moins un deuxième signal de réponse ($S_R$) lors d'une interrogation du corps de capteur (3) avec le signal d'interrogation ($S_I$), et dans lequel l'au moins un premier signal de réponse ($S_R$) et/ou l'au moins un deuxième signal de réponse ($S_R$) sont associés à un ou plusieurs effets de volume de l'au moins un matériau ayant une structure ordonnée (4).

8. Le capteur implantable (1) selon l'une quelconque des revendications précédentes, comprenant en outre un dispositif d'encapsulation qui encapsule partiellement ou entièrement le corps de capteur (3), ledit dispositif d'encapsulation étant de préférence constitué d'un matériau acoustiquement transparent et de manière particulièrement préférée d'un matériau acoustiquement transparent ayant une structure ordonnée (4), et/ou
dans lequel le capteur implantable (1) comprend en outre au moins un dispositif de fixation qui permet ou facilite une fixation du capteur implantable (1) à un tissu dur et/ou à un tissu mou et/ou à un implant, le dispositif de fixation correspondant de préférence à un dispositif de fixation mécanique tel qu'une suture ou des pinces et/ou un dispositif de fixation chimique tel qu'un adhésif.

9. Le capteur implantable (1) selon l'une quelconque des revendications précédentes, dans lequel le capteur implantable (1) est configuré pour une interrogation sans fil, et/ou
dans lequel le capteur implantable (1) est configuré pour une interrogation extracorporelle et/ou une interrogation intracorporelle.

10. Le capteur implantable (1) selon l'une quelconque des revendications précédentes, dans lequel le paramètre est la contrainte et/ou la déformation et/ou une pression et/ou un gonflement et/ou une absorption et/ou une température et/ou un processus biologique tel qu'une inflammation, et/ou
dans lequel le capteur implantable (1) est un capteur de contrainte.

11. Implant (10) comprenant ou constitué d'un capteur implantable (1) selon l'une quelconque des revendications précédentes.

12. Ensemble (100) de détermination d'au moins un paramètre, notamment un paramètre physique, comprenant :

- au moins un capteur implantable (1) comme revendiqué selon l'une quelconque des revendications 1 à 10 ;
- au moins un dispositif d'interrogation (7) configuré pour envoyer au moins un signal d'interrogation ($S_I$) comprenant des ondes acoustiques ;
- au moins un dispositif de réception (8) configuré pour recevoir l'au moins un signal de réponse ($S_R$) ; et
- au moins un dispositif d'évaluation (9) qui est configuré pour déterminer l'au moins un paramètre à partir de l'au moins un signal de réponse ($S_R$) et/ou de l'au moins un signal d'interrogation ($S_i$).

13. Procédé de détermination d'au moins un paramètre, notamment un paramètre physique, à l'aide d'un capteur implantable (1) selon l'une quelconque des revendications 1 à 10 et/ou d'un ensemble (100) selon la revendication 12, le procédé comprenant les étapes suivantes :

- envoyer au moins un signal d'interrogation ($S_I$) au capteur implantable (1) ;
- recevoir au moins un signal de réponse ($S_R$) ; et
- déterminer l'au moins un paramètre à partir de l'au moins un signal de réponse ($S_R$) et/ou de l'au moins un signal d'interrogation ($S_I$).

14. Procédé de fabrication d'un capteur implantable (1) permettant de déterminer au moins un paramètre, notamment un paramètre physique,

dans lequel le procédé comprend l'étape consistant à fournir un corps de capteur (3),

dans lequel le corps de capteur (3) est sensible à l'au moins un paramètre,

dans lequel le corps de capteur (3) est configuré pour produire au moins un signal de réponse ($S_R$) lors d'une interrogation du corps de capteur (3) avec au moins un signal d'interrogation ($S_I$) comprenant des ondes acoustiques,

dans lequel l'au moins un signal de réponse ($S_R$) est associé à l'au moins un paramètre,

**caractérisé en ce que** le corps de capteur (3) comprend ou est constitué d'au moins un matériau ayant une structure ordonnée (4), et

**en ce que** l'au moins un signal de réponse ($S_R$) est associé à un ou plusieurs effets de volume de l'au moins un matériau ayant une structure ordonnée (4),

dans lequel le matériau ayant une structure ordonnée (4) comprend une structure de bande acoustique et/ou une bande interdite acoustique, et dans lequel ladite structure de bande acoustique et/ou de bande interdite acoustique est configurée pour présenter un changement lorsque le corps de capteur (3) est soumis au paramètre, et dans lequel le changement de la structure de bande acoustique et/ou de bande interdite acoustique change le signal de réponse ($S_R$) de manière correspondante, et/ou

dans lequel le matériau ayant une structure ordonnée (4) comprend un réseau et/ou une structure de type réseau, et dans lequel ladite structure et/ou structure de type réseau est déformée lorsque le corps de capteur (3) est soumis au paramètre, et dans lequel la déformation du réseau et/ou de la structure de type réseau change le signal de réponse ($S_R$) de manière correspondante.

15. Produit de programme informatique comprenant des instructions de programme informatique qui, lorsqu'elles sont exécutées dans un processeur du dispositif d'évaluation (9) de l'ensemble (100) selon la revendication 12, amènent le dispositif d'évaluation (9) à effectuer l'étape de détermination de l'au moins un paramètre à partir de l'au moins un signal de réponse ($S_R$) et/ou de l'au moins un signal d'interrogation ($S_I$).

11

11

7; 8

2

2

1; 3; 10

1; 3; 10

**FIG. 1**

**FIG. 2**

x1

x2

x3

10

1; 3; 4

**FIG.3a**

x1

x2

x3

10

1; 3; 4

**FIG. 3b**

EP 4 251 027 B1

FIG. 4

**FIG. 5a**

**FIG. 5b**

**FIG. 5c**

**FIG. 5d**

**FIG. 5e**

FIG. 6

FIG. 7

FIG. 8a

FIG. 8b

FIG. 9a

FIG. 9b

**FIG. 10**

Band structure : y strain - rectangular unit cell

**FIG. 11**

frequency 0 -36 MHz: y axis strain, plane wave (1,1)

FIG. 12

frequency 0 -10 MHz: y axis strain, plane wave (1,1)

FIG. 13a

frequency 0 -10 MHz: y axis strain, plane wave (1,1)

~50kHz

epsilon=0
epsilon=0.01
epsilon=0.02
epsilon=0.03
epsilon=0.04
epsilon=0.05

FIG. 13b

frequency 13 -18 MHz: y axis strain, plane wave (1,1)

FIG. 14a

FIG. 14b

frequency 13 -18 MHz: y axis strain, plane wave (1,0)

FIG. 15

freq(14)=14.3 MHz Total acoustic pressure field (Pa)

FIG. 16a

freq(14)=14.3 MHz Total acoustic pressure field (Pa)

FIG. 16b

freq(14)=14.3 MHz Total acoustic pressure field (Pa)

**FIG. 16c**

freq(14)=14.3 MHz Total acoustic pressure field (Pa)

**FIG. 16d**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20200015678 A1
- US 10595771 B2
- US 10105103 B2
- US 2020359897 A1
- US 2005049499 A1

**Non-patent literature cited in the description**

- Ultrasonic measurements with micromembranes. **BÖRNER M. W. et al.** SENSORS AND ACTUATORS A: PHYSICAL. ELSEVIER BV, 01 January 1995, vol. 46, 62-65